Europäisches Patentamt

**European Patent Office**

**Office européen des brevets**

(19)

Publication number: **0 002 404**

**A1**

(12) **EUROPEAN PATENT APPLICATION**

(21) Application number: 78400185.1

(22) Date of filing: 16.11.78

(51) Int. Cl.²: **A 61 K 39/02**
**C 12 D 13/04, C 12 K 5/00**

(30) Priority: 28.11.77 US 855491

(43) Date of publication of application:
13.06.79 Bulletin 79 12

(84) Designated contracting states:
BE CH DE FR GB LU NL SE

(71) Applicant: MERCK & CO. INC.
126, East Lincoln Avenue
Rahway New Jersey 07065(US)

(72) Inventor: Carlo, Dennis John
195 West Lincoln Avenue
Rahway New Jersey 07065(US)

(72) Inventor: Nolstadt, Karl Heinz
91 Stonehenge Terrace
Clark New Jersey 07066(US)

(72) Inventor: Stoudt, Thomas Henri
857 Village Green
Westfield New Jersey 07090(US)

(72) Inventor: Walton, Robert Bruce
798 Central Avenue
Rahway New Jersey 07065(US)

(72) Inventor: Zeltner, Johanna Yaple
835 Willow Grove Road
Westfield New Jersey 07090(US)

(74) Representative: Corre, Jacques Dénis Paul et al,
Cabinet Regimbeau 26, Avenue Kléber
F-75116 Paris(FR)

(54) **Pneumococcal vaccine and a process for its preparation.**

(57) A vaccine effective against one or more types of *Streptococcus Pneumoniae,* comprising one or more highly purified capsular polysaccharides of *Streptococcus Pneumoniae* obtained by a two-stage alcohol precipitation, followed by removal of proteins and nucleic acids.

EP 0 002 404 A1

-1-        16129Y

PNEUMOCOCCAL VACCINE AND A PROCESS FOR ITS PREPARATION

The present invention is directed to a polyvalent vaccine for bacterial pneumonia. The vaccine comprises a plurality of capsular polysaccharides from different types of Streptococcus pneumoniae. Each capsular polysaccharide is prepared by growing the organism producing that capsular polysaccharide under controlled pH conditions and replenishing the glucose concentration during the fermentation. The pH is maintained at from about 6.2 to about 7.0 by addition of the required amount of an alkaline reagent such as, for example, $NaHCO_3$, $Na_2CO_3$, NaOH, $KHCO_3$, $K_2CO_3$, KOH, $Mg(OH)_2$, $Ca(OH)_2$ or $Al(OH)_3$. Buffer solutions may also be used to control the pH but are less desirable because of the relatively large volume of buffer solution required. A lyophilized culture is conveniently used as the source of the organism. Following the fermentation, the polysaccharide is obtained by a two stage alcohol precipitation followed by removal of proteins and nucleic acids. In the first alcohol precipitation, a predetermined quantity of water-miscible alcohol is added to a liquid material containing the polysaccharide to precipitate impurities while retaining the poly-saccharide in solution. After removal of the pre-cipitated impurities, a second predetermined quantity of water-miscible alcohol is added. The quantity selected for this precipitation is sufficient to precipitate all or substantially all of the capsular polysaccharide without precipitating a substantially quantity of other material. The liquid material containing the polysaccharide may be a fermentation broth in which the desired type of Streptococcus pneumoniae has been grown. While any water-miscible alcohol may be used in the precipitation, preferably the alcohol will have from one to two carbon atoms.

The precipitated polysaccharide is resuspended in liquid medium and treated to remove proteinaceous material and nucleic acids. This may be effected by digesting the liquid medium containing the capsular polysaccharide with an enzyme such as trypsin or nucleases such as ribonuclease or deoxyribonuclease. Alternatively, the liquid material containing the capsular polysaccharide may be treated with a cationic surfactant such as cetrimonium bromide (N,N,N-Trimethyl-1-hexadecanaminium bromide). If necessary, protein fragments, e.g., peptides and nucleic acid fragments may be removed, by appropriate treatment, e.g., by dialysis. The highly purified capsular sccharide of the desired type of Streptococcus pneumoniae is then recovered by precipitation with a water-miscible alcohol. Preferably the alcohol is from 2 to 3 carbon atoms.

The quantity of alcohol or cetrimonium bromide required in the foregoing procedures is determined by a preliminary probe. Two milliliter portions, as many as there are fractions to be tested, of whole broth (or other aqueous solution) are dispensed into 16 X 25 mm disposable culture tubes. The fractions to be investigated are usually taken at 5% intervals over a range $\pm$ 10-15% bracketting the expected precipitation concentration experienced previously on a particular type. For example, if expected interval is 40-45% the following fractions should be examined: 0-35%, 30-35%, 35-40%, 40-45%, 45-50%, and 50-55%.

While agitating on a tube stirrer, alcohol is added to the lower level of each range. Agitation is necessary to avoid local overconcentration which will result in premature precipitation. All the tubes are centrifuged on a desk top clinical centrifuge (7-10,000 rpm) for 10 minutes.

The supernatant liquid is decanted into 16 X 125 ml tubes and alcohol is added, again with agitation, to the upper limit of each fraction. All pellets except the first (which represents material in the 0-low limit of range) are discarded.

The tubes are again centrifuged for 10 minutes and the supernatant liquid is discarded. The pellets in each tube now represent material precipitated in the individual alcohol fractions. Each pellet is dispersed (polysaccharide extracted) in 2 ml of Dulbecco's PBS (1x). The resulting suspension is cleared of insoluble matter with a Swinnex-25 filter (Millipore) mounted on a disposable syringe. A portion of the filtrate from each fraction is diluted 1:2 and 1:4 with PBS. The fractions are now ready for probing.

Antiserum as supplied is initially diluted 1:10 with PBS (1x), and a portion (ca 1.2 ml) of this further diluted 1:6.7; enough to evaluate all the fractions in a particular probe. In general, these dilutions hold. However, an occasional lot of antiserum has been found to be less "potent" and more concentrated dilutions have to be made. This cannot be predicted until experience with a particular lot is gained. 0.2 $Ml$ of diluted antiserum is charged to a series of 10 X 75 ml disposable culture tubes - three for each fraction interval.

Each tube containing antiserum is charged with 10 μl (for ethanol probes) of the fractions to be pooled at the three concentrations (as is, 1:2, 1:4). 5μl is used for isopropanol probes. The tubes are agitated and left to stand 15 minutes - 1 hour. For convenience they are usually arranged in the following network.

|            | 0-30 | 30-35 | 35-40 | 40-45 | 45-50 | 50-55 |
|------------|------|-------|-------|-------|-------|-------|
| Undiluted  | 0    | 0     | C     | 0     | 0     | 0     |
| 1:2        | 0    | 0     | 0     | 0     | 0     | 0     |
| 1:4        | 0    | 0     | 0     | 0     | 0     | 0     |

A positive precipitin reaction is indicated by a distinct opaqueness or an actual precipitate. The desired polysaccharide may be split over two adjacent fractions, or over three fractions where the center one gives a more distinct precipitin reaction. Both of these are positive tests and process alcohol or cetavlon fractionation range is selected based on these results.

There is an optimum concentration for the precipitin reaction, and this is the reason for the three different dilutions. An excess of antibody may result in no precipitate, while an antigen excess often indicates positive results over a large range, sometimes with a weak reaction between two stronger ones. In this case the weak reaction is actually the strongest positive and this can be seen in the more dilute (in antigen) system.

All Streptococcus pneumoniae type designations used in the present application are US type designations rather than Danish type designations. ATCC accession numbers for the various types are as follows:

| Type | ATCC Accession Number |
|------|----------------------|
| 1    | 6301 |
| 2    | 6302 |
| 3    | 6303 |
| 4    | 6304 |
| 6    | 6306 |
| 8    | 6308 |
| 9    | 6309 |
| 10   | 6310 |
| 12   | 6312 |
| 14   | 6314 |
| 15   | 6315 |
| 17   | 6317 |
| 19   | 6319 |
| 20   | 6320 |
| 23   | 6323 |
| 25   | 6325 |
| 51   | 10351 |
| 56   | 10356 |
| 57   | 10357 |
| 72   | 10372 |
| 73   | 10373 |

A vaccine may be prepared by incorporating a purified capsular polysaccharide into a suitable physiologically acceptable medium such as, for example, saline, water for injection or phosphate buffered saline. Polyvalent vaccines may be prepared by incorporating two or more of the purified capsular polysaccharides into a suitable physiologically acceptable medium.

A detailed description of the fermentation process for production of pneumococcal polysaccharide follows.

A lyophilized "L" tube culture is suspended in 1 ml of Difco Heart Infusion Broth (HIB broth) and 0.2 ml is spread on a rabbit blood agar plate (HIB). After approximately 18 hours incubation at 37°C, the growth on the plate is resuspended in 5 ml of HIB broth and 1 ml of the suspension is transferred into 100 ml of rabbit blood liquid medium and incubated as a stationary culture for 18 hours at 37°C. After incubation, the culture is examined microscopically and streaked on YED and HIB plates to check for purity. The 100 ml of culture is stored in a refrigerator at 4°C for a maximum of 1 week.

Twenty ml of the foregoing culture is used to inoculate 1 liter of inoculum medium in a 2-liter Erlenmeyer flask with sidearm for addtion of sodium bicarbonate. The flask is incubated stationary at 37°C until the specifications for the particular type are satisfied. The pH of the fermentation is controlled (6.2-7.0) by the addition of 12% sodium bicarbonate using phenol red in the medium as an indicator. A final sample is examined microscopically and an agglutination test performed to check purity and type. If the resulting culture is not used immediately, it may be stored for 3-4 hours at 4°C. It is preferred, however, to use the culture immediately in the following step.

One liter of the foregoing stage is used to inoculate a 14-liter fermentor containing 9 liters of seed medium. The batch is incubated at 37°C with mild agitation (100 rpm) without air flow. The course of the fermentation is monitored by O.D., pH and glucose determinations. When a specific O.D. is reached, the batch is examined microscopically, an agglutination reaction performed, and a sample plated on YED and HIB plates to examine for purity. The pH is adjusted during fermentation (6.2-7.0) using 10% NaOH. If desired a portion of this culture may be used to inoculate a larger fermentor using the foregoing procedure.

When the batch is terminated, the culture is plated on YED and HIB plates, examined for purity by Gram stain, and identified as to type by the agglutination reaction. The broth is then inactivated by harvesting into liquified phenol (89%) to a final concentration of 1%. After 2 hours in phenol, the broth is released for recovery of product. A sample is removed and plated on HIB to check for viability.

The following culture media are used in the fermentation processes.

1.  YED Plates

| | |
|---|---|
| Yeast Extract | 10 gm per liter |
| Dextrose | 10 gm per liter |
| Agar | 20 gm per liter |

2.  HIB Broth

| | |
|---|---|
| Heart Infusion Broth | 25 gm per liter |

3.  Agar Rabbit Blood Medium (HIB)

| | |
|---|---|
| Heart Infusion Broth | 25 gm per liter |
| Agar | 20 gm per liter |

The above is autoclaved for 20 minutes and cooled to 50°C and 10% defibrinated rabbit blood is added.

4.  Rabbit Blood Liquid Medium

Same as above except agar omitted.

5.  Inoculum Medium - 2 liters

| | |
|---|---|
| HySoy - Humko Sheffield | 20 gm |
| Amberex 1003 | 10 gm |
| NaCl | 5 gm |
| $K_2HPO_4$ | 2.5 gm |
| Phenol red | 10 gm |
| Distilled $H_2O$  brought up to 900 ml | |

16129IA

5. <u>Inoculum Medium - 2 liters</u> (cont'd)

The pH is adjusted to 7.2 and the medium autoclaved for 25 minutes at 121°C.

|  |  |
|---|---|
| Glucose | 25 gm |

The glucose is autoclaved separately in 100 ml distilled $H_2O$ for 20 minutes and added asceptically to above components.

6. <u>14 Liter - Seed Medium for Type 20 and Production Medium for All Other Types</u>

|  |  |
|---|---|
| Hysoy - Humko Sheffield | 180 gm |
| Amberex 1003 | 90 gm |
| NaCl | 45 gm |
| $K_2HPO_4$ | 22.5 gm |
| Phenol red | 90 mg |
| UCON LB 625 8% solution (pre-sterilized for 1 hour) | 40 ml |
| Distilled $H_2O$  brought up to | 8 liters |

The pH is adjusted to 7.2 before sterilization.  The above components are autoclaved together for 90 minutes at 121°C.

|  |  |
|---|---|
| Glucose | 225 gm |
| Distilled $H_2O$  brought up to | 1 liter |

The glucose is autoclaved separately for 25 minutes at 121°C and added asceptically to the above components.

7. <u>Macroscopic Slide Agglutination Identification Test</u>

  a.  A loopful of specific Pneumococcus antiserum (specific type)  is placed on a slide.

  b.  A loopful of <u>Streptococcus pneumoniae</u> (specific type) from the batch is transferred to the drop of antiserum and mixed.

  c.  A positive test is observed if an agglutination of the cells occurs with specific antiserum.

8. <u>Procedure to Detect Viability</u>

A 0.5 ml sample of broth to be checked for viable organisms is spread on Heart Infusion Blood agar.  The plates are incubated at 37°C for 48 hours.  No visible signs of growth after 48 hours represents negative viability.

Pneumococcal polysaccharide type 19 is reported in the literature to consist of L-rhamnose, D-glucose, 2-acetamido-2-deoxy-D-mannose and phosphate in the approximate molar ratio of 2:1:1:1 (Miyazaki et al., Carbohydrate Research, Vol. 16, pp. 153-159, 1971). Based on this report, the theoretical quantity of L-rhamnose in type 19 is 43% and the theoretical quantity of D-glucose is 21.9%. The type 19 polysaccharide product obtained according to the present invention, however, contains in parts by weight about 15-20% L-rhamnose and about 5.5-8% D-glucose. It has also been found to contain about 8-11% 2-acetamido-2-deoxy-D-mannose (N-acetyl mannosamine).

Pneumococcal polysaccharide type 23 is reported in the literature to consist of rhamnose, glucose and galactose in the molar ratios of 5:2:2 (Heidelberger et al., Journal of Immunology, Vol. 99, pp. 794-796, 1967). Based on such ratios the theoretical quantity of L-rhamnose is 59.5%. Larm et al., in Advances in Carbohydrate Chemistry and Biochemistry, Tipson & Horton, editors, Academic Press, 1967, p. 314, indicate that pneumococcal polysaccharide type 23 contains 35% L-rhamnose, 31% D-galactose and 32% D-glucose. The type 23 polysaccharide product obtained according to the present invention, however, contains rhamnose, galactose and glucose in the molar ratios of 2:1:1 and contains in parts by weight about 31-38% L-rhamnose, about 15-18% D-galactose and about 15-18% D-glucose.

Pneumococcal polysaccharide type 25 is reported to contain galacturonic acid as its uronic acid (Heidelberger, Immunochemistry of Bacterial Polysaccharides, in Research in Immunochemistry and Immunobiology, Vol. 3, pp. 1-40, Kwapinski & Day, Eds., University Park Press, Baltimore, 1973).

The type 25 polysaccharide product obtained according to the present invention contains in parts by weight about 2-4% fucose, about 2-4% mannose, about 15-19% galactose, about 1.8-4% D-glucose, about 7-9% galactosamine, about 15-19% glucosamine, and about 5-7% of uronic acid.

The pneumococcal polysaccharide type 72 product obtained according to the present invention contains in parts by weight about 5.5-8% rhamnose, about 3-6.5% fucose, about 2.7-4.8% mannose, about 17-22% D-galactose, about 11.5-16.5% galactosamine and about 27-32% glucosamine.

The pneumococcal polysaccharide type 73 product obtained according to the present invention contains in parts by weight about 1.8-3.5% fucose, about 2-5% mannose, about 14-19% D-galactose, about 12-15% galactosamine and about 29-34% glucosamine.

The foregoing carbohydrate analyses for the types 19, 23, 25, 72 and 73 polysaccharide products of the present invention is determined by gas chromatography.

-11-

The following examples illustrate the present invention without, however, limiting the same thereto. All temperatures are expressed in degrees Celsius.

## EXAMPLE 1

### Pneumococcal Polysaccharide Type 1

A culture of Streptococcus pneumoniae type 1 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1 % (vol/vol) concentration to kill the bacteria.

An ethanol probe is run on a sample of the phenolized whole broth to determine the ethanol range of polysaccharide precipitation. The range for this lot is from 38 to 52%.

With agitation and at room temperature 3.68 liters of ethanol are added slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 2.82 liters of ethanol are added slowly to the supernatant liquid and the resulting mixture aged 4 hours with continuing agitation. The mixture is then centrifuged at 15,000 rpm in 4" Sharples centrifuge to yield a clear supernatant liquid which is discarded. The crude polysaccharide solids are suspended in 0.09 liter of 3% sodium acetate solution. The pH is adjusted to 6.5 with glacial acetic acid and stirred for 4 hours to assure solubilization of the polysaccharide.

An isopropanol probe is run on a sample of the polysaccharide-containing liquid to determine optimum isopropanol range of polysaccharide precipitation. The range for this lot is from 20 to 30%.

With agitation 22.5 ml of isopropanol are added slowly and the resulting mixture aged for 4 hours with continuing agitation. The mixture is preclarified at 15,000 rpm in a 4" Sharples centrifuge. Clarification is completed in a K-2 ultracentrifuge at 25,000-30,000 rpm. The solids are discarded. With agitation an additional 16 ml of isopropanol are added to the supernatant liquid and the resulting mixture allowed to age 4 hours with agitation. The crude polysaccharide precipitate is recovered by centrifuging at 15,000 rpm in a 4" Sharples centrifuge and discarding the supernatant liquid.

The crude polysaccharide is suspended in 90 ml of 3% sodium acetate. The pH is adjusted to 6.5 with glacial acetic acid and 65 mg $MgSO_4$, 2.7 mg ribonuclease and 2.7 mg deoxyribonuclease are added. The solution is digested with agitation at 37° for 3 hours and cooled to 0-5°.

A cetrimonium bromide probe is run on a sample of the liquid to determine the optimum cetavlon range of polysaccharide precipitation. Within this lot the range is from 33-52%. With agitation 59 ml of a 1% aqueous cetrimonium bromide solution is added and the resulting mixture aged for 4 hours. The mixture is clarified in a K-2 ultracentrifuge at 25,000-30,000 rpm. The solids are rpm. The solids are discarded and an additional 96.3 ml of a 1% aqueous cetrimonium bromide solution is added to the supernatant liquid and the resulting mixture aged for 4 hours with agitation. The crude polysaccharide precipitate is recovered by centrifugation at 15,000 rpm in a 4" Sharples centrifuge and discarding the supernatant liquid.

The precipitate is dissolved in 90 ml of a 20% sodium acetate solution and the pH adjusted to 6.5 with glacial acetic acid. Isopropanol, 90 ml, is added with agitation, the mixture is stirred for 2 hours and allowed to settle several hours until a gummy precipitate has separated to the bottom. The entire supernatant liquid is decanted and the precipitate triturated in a Waring blender with about 40 ml of absolute ethanol until a white powder is obtained. The powder is recovered by filtering on a Buchner funnel (Whatma No. 2 paper) washing with 2 X 5 cc of absolute ethanol and 2 X 5 cc acetone. The washed powder is dried at about 27" Hg and room temperature for 12-16 hours to yield about 0.35 g of the purified polysaccharide.

## EXAMPLE 2

### Pneumococcal Polysaccharide Type 2

A culture of Streptococcus pneumoniae type 2 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1 % (vol/vol) concentration to kill the bacteria.

An ethanol probe is run on a sample of the phenolized whole broth to determine the ethanol range of polysaccharide precipitation. The range for this lot is from 40 to 55%.

With agitation and at room temperature 4.0 liters of ethanol are added slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 3.33 liters of ethanol are added slowly to the supernatant liquid and the resulting mixture aged 4 hours with continuing agitation. The mixture is then centrifuged at 15,000 rpm in 4" Sharpless without precipitating a substantial quantity of other material. The liquid material containging the polysaccharide may be a fermentation broth in which the desired type of Streptococcus pneumoniae has been grown. While any water-miscible alcohol may be used in the precipitation, preferably the alcohol will have from one to two carbon atoms.

The precipitated polysaccharide is resuspended in liquid medium and treated to remove proteinaceous material and nucleic acids. This may be effected by digesting the liquid medium containing the capsular polysaccharide with an enzyme such as trypsin or neculeases such as ribonuclease or deoxyribonuclease. Alternatively, the liquid material containging the capsular polysaccharide may be treated with a cationic surfactant such as cetrimonium bromide (N,N,N-trimethyl-1-hexadecanaminium bromide). If necessary, protein fragments, e.g., peptides and nucleic acid fragments may be removed, by appropriate treatment, e.g., by dialysis. The highly purified capsular saccharide of the desired type of Streptococcus pneumoniae is then recovered by precipitation with a water-miscible.alcohol. Preferably the alcohol is from 2 to 3 carbon atoms.

The quantity of alcohol or cetrimonium bromide required in the foregoing procedures is determined by a preliminary probe. Two milliliter portions, as many as there are fractions to be tested, of whole broth (or other aqueous solution) are dispensed into 16 X 25 cm disposable culture tubes. The fractions to be investigated are usually taken at 5% intervals over a range ± 10-15% bracketting the expected precipitation concentration experienced previously on a centrifuge to yield a clear supernatant liquid which is discarded. The crude polysaccharide solids are suspended in 0.6 liters of 1% sodium acetate solution and the mixture stirred for 4 hours to assure solubilization of the polysaccharide.

The mixture is preclarified at 15,000 rpm in a 4" Shaples centrifuge. Clarification is completed in a K-2 ultracentrifuge at 25,000-30,000 rpm. The solids are discarded.

The pH of the supernatant liquid is adjusted to 8.0 with 50% NaOH, 12 mg of trypsin are added, and the solution digested at 37° for 90 minutes. The mixture is cooled to 20-25° and the pH adjusted to 6.5 with glacial acetic acid.

The digested solution is cooled to 0-5°, and 7.2 g cetrinonium bromide are added. The cetrinonium bromide solution is aged for 4 hours at 0-5° and centrifuged in a K-2 ultracentrifuge at 25,000-30,000 rpm. The solids are discarded.

Sodium acetate, 120 g, is added to the liquid followed by 0.6 liter of ethanol. The mixture is agitated for 2 hours and the precipitate recovered by centrifugation in 4" Sharples centrifuge at 15,000 rpm. The precipitate is triturated with 10-20 ml of 66% ethanol/water and recovered by filtration on a Buchner funnel.

The crude polysaccharide is suspended in 300 ml of 3% sodium acetate solution and the pH is adjusted to 6.5 with glacial acetic acid and the mixture is stirred for 4 hours to assure solubilization of the polysaccharide.

An isopropanol probe is run on a sample of the liquid to determine the optimum isopropanol range of polysaccharide precipitation. With this lot the range is from 28-42% (vol/vol). With agitation 117 ml of isopropanol are added and the resulting mixture aged for 4 hours. The mixture is preclarified in a 4" Sharples centrifuge at 15,000 rpm. Clarification is completed in a K-2 ultracentrifuge at 25,000-30,000 rpm. The solids are discarded and an additional 100 ml of isopropanol is added to the supernatant liquid and the resulting mixture aged for 4 hours with agitation. The crude polysaccharide precipitate is recovered by centrifugation at 15,000 rpm in a 4" Sharples centrifuge and discarding the supernatant liquid.

The precipitate is triturated in a Waring blender with about 40 ml of absolute ethanol until a white powder is obtained. The powder is recovered by filtering on a Buchner funnel (Whatman No. 2 paper) washing with 2 X 5 cc cc of absolute ethanol and 2 X 5 cc acetone. The washed powder is then dried at 27" Hg and room temperature for 12-16 hours to yield about 4.6 g of the purified polysaccharide.

## EXAMPLE 3

### Pneumococcal Polysaccharide Type 3

A culture of Streptococcus pneumoniae type 3 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1 % (vol/vol) concentration to kill the bacteria.

An ethanol probe is run on a sample of the phenolized whole broth to determine the ethanol range of polysaccharide precipitation. The range for this lot is from 28 to 45%.

With agitation and at room temperature 2.33 liters of ethanol are added slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 2.58 liters of ethanol are added slowly to the supernatant liquid and the resulting mixture aged 4 hours with continuing agitation. The mixture is then centrigued at 15,000 rpm in 4" Sharples centrifuge. The supernatant liquid is discarded and the crude polysaccharide solids are suspended in 1.8 liters of 3% sodium acetate solution with the aid of a high shear mixer. The pH is adjusted to 6.5 with glacial acetic acid and stirred for 4 hours to assure solubilization of the polysaccharide.

An isopropanol probe is run on a sample of the polysaccharide-containing liquid to determine optimum isopropanol range of polysaccharide precipitation. The range for this lot is from 18 to 30% (vol/vol).

With agitation 0.395 liter of isopropanol is added slowly and the resulting mixture aged for 4 hours with continuing agitation. The mixture is preclarified at 15,000 rpm in a 4" Sharples centrifuge. Clarification is completed in a K-2 ultracentrifuge at 25,000-30,000 rpm. The solids are discarded. With agitation an additional 0.376 liter of isopropanol is added slowly to the supernatant liquid and the resulting mixture allowed to age 4 hours with agitation. The crude polysaccharide precipitate is recovered by centrifuging at 15,000 rpm in a 4" Sharples centrifuge and discarding the supernatant liquid.

The crude polysaccharide is suspended with the aid of a high shear mixer in 1.0 liter of pH 7.0, 0.05 M phosphate buffer. The pH is readjusted to 7.0

0002404

with glacial acetic acid and 60 mg MgCl$_2$, 0.6 mg ribonuclease and 0.6 mg deoxyribonuclease are added.  The solution is digested with agitation at 37° for 60 minutes.  The pH is adjusted to 8.0 with 50% NaOH, 18 mg of trypsin are added, and the digestion is continued at 37° for an additional 90 minutes.  The mixture is cooled to 20-25° and the pH adjusted to 6.5 with glacial acetic acid.

The digested solution is diafiltered in a Romicon hollow filter ultrafiltration membrane until the electrical conductivity of the permeate reaches a constant value.  The reservoir is kept at constant volume with pyrogen free water.  The retentate which contains the polysaccharide is drained from the system and the volume restored to 1.0 liter with system washes.

Sodium acetate, 30 g, is added to the solution of the polysaccharide-containing liquid and the pH is adjusted to 6.5 with glacial acetic acid.  With agitation 2.0 liters of isopropanol are added and the resulting mixture stirred for 15 minutes and settled for several hours until a gummy precipitate has separated to the bottom.  The entire supernatant liquid is decanted and the precipitate is triturated in a Waring blender with about 40 ml of absolute ethanol until a white powder is obtained. The powder is recovered by filtering on a Buchner funnel (Whatman No. 2 paper) washing with 2 X 5 cc of absolute ethanol and 2 X 5 cc acetone.  The washed powder is then dried at 27" Hg and room temperature for 12-16 hours to yield about 2.76 g of the purified poly-saccharide.

## EXAMPLE 4

## Pneumococcal Polysaccharide Type 4

A culture of Streptococcus pneumoniae type 4 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1 % (vol/vol) concentration to kill the bacteria.

An ethanol probe is run on a sample of the phenolized whole broth to determine the ethanol range of polysaccharide precipitation. The range for this lot is from 68 to 80% (vol/vol).

With agitation and at room temperature 12.75 liters of ethanol are added slowly slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 11.25 liters of ethanol are added slowly to the supernatant liquid and the resulting mixture aged 4 hours with continuing agitation. The mixture is then centrifuged at 15,000 rpm in 4" Sharples centrifuge. The supernatant liquid is discarded and the crude polysaccharide solids are suspended in 0.6 liter of 3% sodium acetate solution with the aid of a high shear mixer. The pH is adjusted to 6.5 with glacial acetic acid and stirred for 4 hours to assure solubilization of the polysaccharide.

An isopropanol probe is run on a sample of the polysaccharide-containing liquid to determine optimum isopropanol range of polysaccharide precipitation. The range for this lot is from 42 to 55% (vol/vol).

With agitation 0.434 liter of isopropanol is added slowly and the resulting mixture aged for 4 hours with continuing agitation. The mixture is preclarified at 15,000 rpm in a 4" Sharples centrifuge. Clarification is completed in a K-2 ultracentrifuge at 25,000-30,000 rpm. The solids are discarded. With agitation an additional 0.3 liter of isopropanol is added slowly to the supernatant liquid and the resulting mixture allowed to age 4 hours with agitation. The crude polysaccharide precipitate is recovered by centrifuging at 15,000 rpm in a 4" Sharples centrifuge and discarding the supernatant liquid.

The crude polysaccharide is suspended with the aid of a high shear mixer in 0.3 liter of pH 7.0, 0.05 M phosphate buffer. The pH is readjusted to 7.0 with glacial acetic acid and 60 mg $MgCl_2$, 0.6 mg ribonuclease and 0.6 mg deoxyribonuclease are added. The solution is digested with agitation at 37° for 60 minutes. The pH is adjusted to 8.0 with 50% NaOH, 18 mg of trypsin are added, and the digestion is continued at 37° for an additional 90 minutes. The mixture is cooled to 20-25° and the pH adjusted to 6.5 with glacial acetic acid.

The digested solution is diafiltered in a Romicon hollow filter ultrafiltration membrane until the electrical conductivity of the permeate reaches a constant value. The reservoir is kept at constant volume with pyrogen free water. The retentate which contains the polysaccharide is drained from the system and the volume restored to 0.3 liter with system washes.

Sodium acetate, 9 g, is added to the solution of the polysaccharide-containing liquid and the pH is adjusted to 6.5 with glacial acetic acid. With agitation 2.0 liters of isopropanol are added and the resulting mixture stirred for 15 minutes and settled for several hours until a gummy precipitate has separated to the bottom. The entire supernatant liquid is decanted and the precipitate is triturated in a Waring blender with about 40 ml of absolute ethanol until a white powder is obtained. The powder is recovered by filtering on a Buchner funnel (Whatman No. 2 paper) followed by washing with 2 X 5 cc of absolute ethanol and 2 X 5 cc acetone. The washed powder is then dried at 27" Hg and room temperature for 12-16 hours to yield about 1.32 g of the purified polysaccharide.

## EXAMPLE 5

### Pneumococcal Polysaccharide Type 6

A culture of Streptococcus pneumoniae type 6 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1 % (vol/vol) concentration to kill the bacteria.

An ethanol probe is run on a sample of the phenolized whole broth to determine the ethanol range of polysaccharide precipitation. The range for this lot is from 47 to 60% (vol/vol).

With agitation and at room temperature 5.32 liters of ethanol are added slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 3.68 liters of ethanol are added slowly to the supernatant liquid and the resulting mixture aged 4 hours with continuing agitation. The mixture is then centrigued at 15,000 rpm in 4" Sharples centrifuge. The supernatant liquid is discarded and the crude polysaccharide solids are suspended in 0.6 liter of 3% sodium acetate solution with the aid of a high shear mixer. The pH is adjusted to 6.5 with glacial acetic acid and stirred for 4 hours to assure solubilization of the polysaccharide.

An isopropanol probe is run on a sample of the polysaccharide-containing liquid to determine optimum isopropanol range of polysaccharide precipitation. The range for this lot is from 35 to 47% (vol/vol).

With agitation 0.323 liter of isopropanol is added slowly and the resulting mixture aged for 4 hours with continuing agitation. The mixture is preclarified at 15,000 rpm in a 4" Sharples centrifuge. Clarification is completed in a K-2 ultracentrifuge at 25,000-30,000 rpm. The solids are discarded. With agitation an additional 0.21 liter of isopropanol is added slowly to the supernatant liquid and the resulting mixture allowed to age 4 hours with agitation. The crude polysaccharide precipitate is recovered by centrifuging at 15,000 rpm in a 4" Sharples centrifuge and discarding the supernatant liquid.

The crude polysaccharide is suspended with the aid of a high shear mixer in 0.3 liter of pH 7.0, 0.05 M phosphate buffer. The pH is readjusted to 7.0 with glacial acetic acid and 60 mg $MgCl_2$, 0.6 mg ribonuclease and 0.6 mg deoxyribonuclease are added. The solution is digested with agitation at 37° for 60 minutes. The pH is adjusted to 8.0 with 50% NaOH, 18 mg of trypsin are added, and the digestion is continued at 37° for an additional 90 minutes. The mixture is cooled to 20-25° and the pH adjusted to 6.5 with glacial acetic acid.

0002404

The digested solution is diafiltered in a Romicon hollow filter ultrafiltration membrane until the electrical conductivity of the permeate reaches a constant value. The reservoir is kept at constant volume with pyrogen free water. The retentate which contains the polysaccharide is drained from the system and the volume restored to 0.3 liter with system washes.

Sodium acetate, 9 g, is added to the solution of the polysaccharide-containing liquid and the pH is adjusted to 6.5 with glacial acetic acid. With agitation 0.6 liter of isopropanol are added and the resulting mixture stirred for 15 minutes and settled for several hours until a gummy precipitate has separated to the bottom. The entire supernatant liquid is decanted and the precipitate is triturated in a Waring blender with about 40 ml of absolute ethanol until a white powder is obtained. The powder is recovered by filtering on a Buchner funnel (Whatman No. 2 paper) washing with 2 X 5 cc of absolute ethanol and 2 X 5 cc acetone. The washed powder is then dried at 27" Hg and room temperature for 12-16 hours to yield about 4.08 g of the purified polysaccharide.

## EXAMPLE 6

### Pneumococcal Polysaccharide Type 8

A culture of Streptococcus pneumoniae type 8 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1 % (vol/vol) concentration to kill the bacteria.

An ethanol probe is run on a sample of the phenolized whole broth to determine the ethanol range of polysaccharide precipitation. The range for this lot is from 23 to 35% (vol/vol).

With agitation and at room temperature 1.79 liters of ethanol are added slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 1.44 liters of ethanol are added slowly to the supernatant liquid and the resulting mixture aged 4 hours with continuing agitation. The mixture is then centrigued at 15,000 rpm in 4" Sharples centrifuge. The supernatant liquid is discarded and the crude polysaccharide solids are suspended in 0.3 liters of 3% sodium acetate solution with the aid of a high shear mixer. The pH is adjusted to 6.5 with glacial acetic acid and the mixture stirred for 4 hours to assure solubilization of the polysaccharide.

The mixture is preclarified at 15,000 rpm in a 4" Sharples centrifuge. Clarification is completed in a K-2 ultracentrifuge at 25,000-30,000 rpm. The solids are discarded.

The pH of the supernatant liquid is adjusted to 7.0 with glacial acetic acid and 60 mg $MgCl_2$, 0.6 mg ribonuclease and 0.6 mg deoxyribonuclease are added. The solution is digested with agitation at 37° for 60 minutes. The pH is adjusted to 8.0 with 50% NaOH, 12 mg of trypsin are added, and the digestion is continued at 37° for an additional 90 minutes. The mixture is cooled to 20-25° and the pH adjusted to 6.5 with glacial acetic acid.

The digested solution is diafiltered in a Romicon hollow filter ultrafiltration membrane until the electrical conductivity of the permeate reaches a constant value. The reservoir is kept at constant volume with pyrogen free water. The retentate which contains the polysaccharide is drained from the system and the volume restored to 1.0 liter with system washes.

The pH of the polysaccharide-containing liquid is adjusted to pH 6.5 with glacial acetic acid and an isopropanol probe is run on a sample of the liquid to determine the optimum isopropanol range of polysaccharide precipitation. With this lot the range is from 32-47%. With agitation 0.141 liter of isopropanol is added and the resulting mixture aged for 4 hours. The mixture is preclarified in a 4" Sharples centrifuge at 15,000 rpm. Clarification is completed in a K-2 ultracentrifuge at 25,000-30,000 rpm. The solids are discarded and an additional 0.125 liter of isopropanol is added to the supernatant liquid and the resulting mixture aged for 4 hours with agitation. The crude polysaccharide precipitate is recovered by centrifugation at 15,000 rpm in a 4" Sharples centrifuge and discarding the supernatant liquid.

With the aid of a high shear mixer the crude polysaccharide is suspended in 0.3 liter of sodium acetate solution and the pH adjusted to 6.5 with glacial acetic acid. Isopropanol, 0.6 liter, is added with agitation, and the mixture is stirred for 2 hours and settled several hours until a gummy precipitate has separated to the bottom. The entire supernatant liquid is decanted and the precipitate is triturated in a Waring blender with about 40 ml of absolute ethanol until a white powder is obtained. The powder is recovered by filtering on a Buchner funnel (Whatman No. 2 paper) washing with 2 X 5 cc of absolute ethanol and 2 X 5 cc acetone. The washed powder is then dried at 27" Hg and room temperature for 12-16 hours to yield about 0.72 g of the purified polysaccharide.

the aid of a high shear mixer. The pH is adjusted to 6.5 with glacial acetic acid and stirred for 4 hours to assure solubilization of the polysaccharide.

An isopropanol probe is run on a sample of the polysaccharide-containing liquid to determine optimum isopropanol range of polysaccharide precipitation. The range for this lot is from 32 to 47% (vol/vol).

With agitation 0.282 liter of isopropanol is added and the resulting mixture aged for 4 hours with continuing agitation. The mixture is preclarified at 15,000 rpm in a 4" Sharples centrifuge. Clarification is completed in a K-2 ultracentrifuge at 25,000-30,000 rpm. The solids are discarded. With agitation an additional 0.25 liter of isopropanol is added to the supernatant liquid and the resulting mixture allowed to age 4 hours with agitation. The crude polysaccharide precipitate is recovered by centrifuging at 15,000 rpm in a 4" Sharples centrifuge and discarding the supernatant liquid.

The crude polysaccharide is suspended with the aid of a high shear mixer in 0.6 liter of 0.9% sodium chloride. The pH is adjusted to 8.0 with 50% NaOH, 12 mg of trypsin are added, and the solution is digested at 37° for 90 minutes. The mixture is cooled to 20-25° and the pH adjusted to 6.5 with glacial acetic acid.

The solution is cooled to 0-5°, 285 g of ammonium sulfate are added, and the mixture aged at 0-5° for 4 hours. The precipitate which forms is collected by centrifuging at 15,000 rpm in a 4" Sharples centrifuge. The supernatant liquid is discarded.

## EXAMPLE 7

### Pneumococcal Polysaccharide Type 9

A culture of Streptococcus pneumoniae type 9 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1% (vol/vol) concentration to kill the bacteria.

An ethanol probe is run on a sample of the phenolized whole broth to determine the ethanol range of polysaccharide precipitation. The range for this lot is from 45 to 60% (vol/vol).

With agitation and at room temperature 4.9 liters of ethanol are added slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 4.1 liters of ethanol are added slowly to the supernatant liquid and the resulting mixture aged 4 hours with continuing agitation. The mixture is then centrigued at 15,000 rpm in 4" Sharples centrifuge. The supernatant liquid is discarded and the crude polysaccharide solids are suspended in 0.6 liter of 3% sodium acetate solution with

The precipitate is dissolved in 0.3 liter of pyrogen free water and the solution diafiltered in a Romicon hollow filter ultrafiltration membrane until the electrical conductivity of the permeate reaches a constant value.  The reservoir is kept at constant volume with pyrogen free water.  The retentate which contains the polysaccharide is drained from the system and the volume restored to 0.3 liter with system washes.

Sodium acetate, 9 g, is added to the solution of the polysaccharide-containing liquid and the pH is adjusted to pH 6.5 with glacial acetic acid.  With agitation 0.6 liter of isopropanol is added and the resulting mixture stirred for 2 hours and settled for several hours until a gummy precipitate has separated to the bottom.  The entire supernatant liquid is decanted and the precipitate is triturated in a Waring blender with about 40 ml of absolute ethanol until a white powder is obtained. The powder is recovered by filtering on a Buchner funnel (Whatman No. 2 paper) washing with 2 X 5 cc of absolute ethanol and 2 X 5 cc acetone.  The washed powder is then dried at 27" Hg and room temperature for 12-16 hours to yield about 1.68 g of the purified polysaccharide.

## EXAMPLE 8

### PNEUMOCOCCAL POLYSACCHARIDE TYPE 10

A culture of Streptococcus pneumoniae type 10 received from the American Type Culture Collection (ATCC Number 6310), and preserved as a lyophilized culture is suspended in 1 ml of Difco Heart Infusion Broth (HIB broth) and 0.2 ml is spread on a rabbit blood agar plate. After 18 hours incubation at 37°C, the growth on the plate is resuspended in 5 ml of HIB broth and 1 ml of the suspension is transferred into 100 ml of rabbit blood liquid medium and incubated as a stationary culture for 18 hours at 37°C. After incubation the culture is examined microscopically and streaked on YED and HIB plates to check for purity. The 100 ml of culture is stored in the refrigerator at 40°C. Ten ml of the culture of the incubated broth is used to inoculate 1 liter of inoculum medium in a 2-liter Erlenmeyer flask. The flask is incubated stationary for 18 1/2 hours at 37°C. The pH of the fermentation is controlled (6.4 - 7.0) by the addition of sodium bicarbonate (12%) until an O.D. (optical density) of 2.2 is reached. The 1 liter fermentation utilizes 80 ml of sodium bicarbonate in reaching the above mentioned O.D. Before inoculating the next stage, a sample is plated on YED and HIB plates to check purity. A sample is also examined microscopically and an agglutination test performed.

The one liter of fermentation broth from the previous step is used to inoculate a 14-liter fermentor containing 9 liters of production medium. The batch is incubated at 37°C with mild agitation (100 rpm) without air flow. The course of the fermentation is monitored by O.D., pH and glucose determinations.

After the final sodium hydroxide addition, the fermentation is continued for one more hour and then terminated (total time 9 hours). The final O.D. before harvesting is 4.24. The pH is controlled during fermentation (6.2 - 7.0) by using 10% NaOH. a total of 725 ml of NaOH (10%) is utilized. When the batch is terminated, the culture is plated on YED and HIB plates, examined for purity by Gram stain, and identified as to type by the agglutination reaction. The broth is inactivated by harvesting into liquefied phenol (89%) to a final concentration of 1%. After 2 hours in phenol the broth is released for recovery of product. A sample is removed and plated on HIB to check for viability.

An ethanol probe is run on a sample of the phenolized whole broth to determine the ethanol range of polysaccharide precipitation. The range for this lot is from 48 to 66% (vol/vol). The alcohol (95% denatured ethanol) is added to 10 liters of the broth at a rate of 100 ml/minute with constant stirring until the ethanol concentration is 48% (vol/vol). The broth is then cleared by centrifugation at 30,000 rpm in a Sharpless centrifuge, Model T-1-P.

To the clear effluent, ethanol is added to 66% (vol/vol) based on the original aqueous system. The precipitate is collected by siphoning off most of the supernatant and by centrifuging the remaining supernatant plus crude polysaccharide in a Sorvall RC5 at 6,000 rpm (6089 xg) for 20 minutes at 15°C.

The pellets are resuspended in 1,000 ml of 3% sodium acetate with the aid of a Waring blender at low speed. The pH is adjusted to 6.5.

16129IA

Isopropanol probing indicates that the polysaccharide precipitates between 38-47% (vol/vol) of isopropanol at room temperature. Therefore, isopropanol is added dropwise with stirring on a thermolyne stir plate, middle setting, to a concentration of 38% (vol/vol). The suspension is then cleared by centrifugation in a Beckman J21 at 14,000 rpm (30,050 xg) at 15°C for 20 minutes. The clear supernatant is diluted with isopropanol to a final concentration of 47% (vol/vol). This results in the settling out of a precipitate which is collected by siphoning the supernatant (yellow, brown color).

The precipitated polysaccharide is resuspended in 1,000 ml of water and the pH is adjusted to 7.0 with concentrated acetic acid. 203 Mg of $MgCl_2$ (1 mM), 8 mg of deoxyribonuclease and 8 mg of ribonuclease are added with stirring. The suspension is incubated in a gyratory water bath, for 90 minutes at 37°C. After incubation, the pH is adjusted to 8.0 with 1 N NaOH, and 49 mg of trypsin is added. The digest is incubated as above.

The pH is adjusted to 7.0 and the digest is dispensed in 1 1/8" dialysis tubing and dialyzed against 14 liters of water with one change of water. The dialysis product, 1080 ml, is recovered and brought to 3% sodium acetate. Isopropanol probing indicates that the purified polysaccharide precipitates between 40-52% (vol/vol) isopropanol at room temperature. The precipitation is performed as in the previous isopropanol precipitation. The polysaccharide which adheres to the bottom of the glass beaker is collected by siphoning off the final supernatant. It is triturated in a Waring blender at high speed with 500 ml of absolute ethanol and is poured onto a medium sinter glass funnel. The polysaccharide is washed with 500 ml of ethanol followed by 250 ml of acetone. Excess solvent is removed by suction and the polysaccharide dried _in vacuo_ over $CaSO_4$. The yield is 7.9 g of pneumococcal polysaccharide type 10.

## EXAMPLE 9

### Pneumococcal Polysaccharide Type 12

A culture of _Streptococcus pneumoniae_ type 12 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1% (vol/vol) concentration to kill the bacteria.

An ethanol probe is run on a sample of the phenolized whole broth to determine the ethanol range of polysaccharide precipitation. The range for this lot is from 42 to 58% (vol/vol).

With agitation and at room temperature 4.35 liters of ethanol are added slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 3.94 liters of ethanol are added slowly to the supernatant liquid and the resulting mixture aged 4 hours with continuing agitation. The mixture is then centrigued at 15,000 rpm in 4" Sharples centrifuge. The supernatant liquid is discarded and the crude polysaccharide solids are suspended in 0.3 liter of 3% sodium acetate solution with the aid of a high shear mixer. The pH is adjusted to 6.5 with glacial acetic acid and stirred for 4 hours to assure solubilization of the polysaccharide.

An isopropanol probe is run on a sample of the polysaccharide-containing liquid to determine optimum isopropanol range of polysaccharide precipitation. The range for this lot is from 32 to 47% (vol/vol).

With agitation 0.141 liter of isopropanol is added and the resulting mixture aged for 4 hours with continuing agitation. The mixture is preclarified at 15,000 rpm in a 4" Sharples centrifuge. Clarification is completed in a K-2 ultracentrifuge at 25,000-30,000 rpm. The solids are discarded. With agitation an additional 0.125 liter of isopropanol is added to the supernatant liquid and the resulting mixture allowed to age 4 hours with agitation. The crude polysaccharide precipitate is recovered by centrifuging at 15,000 rpm in a 4" Sharples centrifuge and discarding the supernatant liquid.

With the aid of a high shear mixer the crude polysaccharide is suspended in 0.3 liter of pH 7.0, 0.05 M phosphate buffer. The pH is readjusted to 7.0 with glacial acetic acid and 60 mg $MgCl_2$, 0.6 mg ribonuclease and 0.6 mg deoxyribonuclease are added. The solution is digested at 37° for 60 minutes. The pH is adjusted to 8.0 with 50% NaOH, 12 mg of trypsin are added, and the digestion is continued at 37° for an additional 90 minutes. The mixture is cooled to 20-25° and the pH adjusted to 6.5 with glacial acetic acid.

The solution is cooled to 0-5°, 144 g of ammonium sulfate are added, and the mixture aged at 0-5°C for 4 hours. The precipitate which forms is collected by centrifuging at 15,000 rpm in a 4" Sharples centrifuge. The supernatant liquid is discarded.

The precipitate is dissolved in 0.3 liter of pyrogen free water and the solution diafiltered in a Romicon hollow filter ultrafiltration membrane until the electrical conductivity of the permeate reaches a constant value. The reservoir is kept at constant volume with pyrogen free water. The retentate which contains the polysaccharide is drained from the system and the volume restored to 0.3 liter with system washes.

Sodium acetate, 9 g, is added to the solution of the polysaccharide-containing liquid and the pH is adjusted to pH 6.5 with glacial acetic acid. With agitation 0.6 liter of isopropanol is added and the resulting mixture stirred for 2 hours and settled for several hours until a gummy precipitate has separated to the bottom. The entire supernatant liquid is decanted and the precipitate is triturated in a Waring blender with about 40 ml of absolute ethanol until a white powder is obtained. The powder is recovered by filtering on a Buchner funnel (Whatman No. 2 paper) washing with 2 X 5 cc of absolute ethanol and 2 X 5 cc acetone. The washed powder is then dried at 27" Hg and room temperature for 12-16 hours to yield about 1.2 g of the purified polysaccharide.

## EXAMPLE 10

### Pneumococcal Polysaccharide Type 14

A culture of Streptococcus pneumoniae type 14 is grown on blood agar for 16 hours at 37°. The cells are collected, and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1 % (vol/vol) concentration to kill the bacteria.

An ethanol probe is run on a sample of the phenolized whole broth to determine the ethanol range of polysaccharide precipitation. The range for this lot is from 38 to 53% (vol/vol).

With agitation and at room temperature 3.7 liters of ethanol are added slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 3.1 liters of ethanol is added slowly to the supernatant liquid and the resulting mixture aged 4 hours with continuing agitation. The mixture is then centrifuged at 15,000 rpm in a 4" Sharples centrifuge. The supernatant liquid is discarded and the crude polysaccharide solid are suspended in 0.5 liters of pyrogen free water and stirred for 4 hours to assure solubilization of the polysaccharide. Twelve liters of a 5% sodium acetate solution are added and the pH is adjusted to 6.5 with glacial acetic acid.

An isopropanol probe is run on a sample of the polysaccharide-containing liquid to determine optimum isopropanol range of polysaccharide precipitation. The range for this lot is from 30 to 42% (vol/vol).

With agitation 0.25 liter of isopropanol is added and the resulting mixture aged for 4 hours with continuing agitation. The mixture is preclarified at 15,000 rpm in a 4" Sharples centrifuge. Clarification is completed in a K-2 ultracentrifuge at 25,000-30,000 rpm. The solids are discarded. With agitation an additional 0.18 liter of isopropanol is added to the K2 supernatant and the resulting mixture allowed to age 4 hours with agitation. The crude polysaccharide precipitate is recovered by centrifuging at 15,000 rpm in a 4" Sharples centrifuge and discarding the supernatant liquid.

The precipitate is dissolved in 0.3 liter of pyrogen free water and the solution is diafiltered in a Romicon hollow filter ultrafiltration membrane until the electrical conductivity of the permeate reaches a constant value. The reservoir is kept at constant volume with pyrogen free water. The retentate which contains the polysaccharide is drained from the system and the volume restored to 0.3 liter with system washes. The solution is clarified in the K-2 ultracentrifuge at 20,000-25,000 rpm.

The clarified solution is cooled to 0-5°. Thirty ml of solution of 6.6 grams of cetavalon in pyrogen free water is added to give a final concentration of 2%. the mixture is aged for 4 hours at 0.5° and clarified in the K-2 ultracentrifuge at 20,000-25,000 rpm. Sixty-six grams of sodium acetate are added to the supernatant liquid and the pH is adjusted to 6.5 with glacial acetic acid.

An isopropanol probe is run on a sample of the suspension to determine the optimum isopropanol range of polysaccharide precipitation. With this lot the range is from 29-40% (vol/vol). With agitation 0.14 liter of isopropanol is added and the resulting mixture aged for 4 hours. The mixture is preclarified in a 4" Sharples centrifuge at 15,000 rpm. Clarification is completed in a K-2 ultracentrifuge at 25,000-30,000 rpm. The solids are discarded and an additional 0.085 liter of isopropanol is charged to the K-2 supernatant and the resulting mixture aged for 4 hours with agitation. The crude polysaccharide precipitate is recovered by centrifugation at 15,000 rpm in a 4" Sharples centrifuge and discarding the supernatant liquid.

The precipitate is triturated in a Waring blender with about 40 ml of absolute ethanol until a white powder is obtained. The powder is recovered by filtering on a Buchner funnel (Whatman No. 2 paper) washing with 2 X 5 cc of absolute ethanol and 2 X 5 cc acetone. The washed powder is then dried at 27" Hg and room temperature for 12-16 hours to yield about 1.1 g of the purified polysaccharide.

## EXAMPLE 11
## PNEUMOCOCCAL POLYSACCHARIDE TYPE 15

A lyophilized culture of Streptococcus pneumoniae type 15 is suspended in 1 ml of Difco Heart Infusion Broth (HIB broth) and 0.2 ml is spread on a rabbit blood agar plate. After 18 hours incubation at 37°C, the growth on the plate is resuspended in 5 ml of HIB broth and 1 ml of the suspension is transferred into 100 ml of rabbit blood liquid medium and incubated as a stationary culture for 18 hours at 37°C. After incubation the culture is examined microscopically and streaked on YED and HIB plates to check for purity. The 100 ml of culture is stored in the refrigerator at 40°C.

Ten ml of the culture of the incubated broth is used to inoculate 1 liter of inoculum medium in a 2-liter Erlenmeyer flask. The flask is incubated stationary for 19 hours at 37°C. The pH of the fermentation is controlled (6.4-7.0) by the addition of sodium bicarbonate (12%) until an O.D. of 2.9 is reached. The 1 liter fermentation utilizes 100 ml of sodium bicarbonate in reaching the above mentioned O.D. Before inoculating the next stage a sample is plated on YED and HIB plates to check purity. A sample is also examined microscopically and an agglutination test performed to check purity and type.

The one liter of fermentation broth from the previous step is used to inoculate a 14-liter fermentor containing 9 liters of production medium. The batch is incubated at 37°C with mild agitation (100 rpm) without air flow. The course of the fermentation is monitored by O.D., pH and glucose determinations.

After the final sodium hydroxide addition, the fermentation is continued for one more hour and then terminated (total time 8 hours). The final O.D. before harvesting is 4.0. The pH is controlled during fermentation (6.2-7.0) by using 10% NaOH. A total of 720 ml of NaOH (10%) is utilized.

When the batch is terminated the culture is plated on YED and HIB plates, examined for purity by Gram stain, and identified as to type by the agglutination reaction. The broth is inactivated by harvesting into liquefied phenol (89%) to a final concentration of 1%. After 2 hours in phenol the broth is released for recovery of product. A sample is removed and plated on HIB to check for viability.

An ethanol probe is run on a sample of the phenolized whole broth to determine the ethanol range of polysaccharide precipitation. The range for this lot is from 63 to 72% (vol/vol). The alcohol (95% denatured alcohol) is added to 10 liters of the broth at a flow rate of 100 ml/minute until the alcohol concentration is 63% (vol/vol). The broth is then centrifuged in a Sharples centrifuge Model T-1-P at 30,000 rpm.

To the clear effluent, 95% denatured alcohol is added to a final concentration of 72% vol/vol based on the original aqueous volume. The suspension is left overnight at room temperature. Since the polysaccharide does not precipitate out, sodium acetate is added to a final concentration of 1%. The crude polysaccharide precipitates out rapidly and is collected by siphoning off the clear supernatant. The polysaccharide is resuspended in 5% sodium acetate.

Isopropanol probing indicates that the polysaccharide precipitates between 40-50% (vol/vol) of isopropanol at room temperature. Therefore, isopropanol is added dropwise with stirring on a thermolyne magnestir, middle setting to a concentration of 40% (vol/vol). The suspension is cleared by centrifugation in a Beckman J21 at 14,000 rpm (~ 30,050 xg) for 20 minutes at 4°C in 250 ml cups. The clear supernatant is brought to 50% isopropanol (vol/vol). The precipitated polysaccharide is collected by siphoning off the supernatant.

The precipitate is resuspended in 1,000 ml of water. 0.203 G of $MgCl_2$ (1 mM), 8 mg of ribonuclease and 7 mg of deoxyribonuclease are added with stirring as in the previous step. The pH is adjusted to 7.0 with concentrated acetic acid and the mixture is incubated for 90 minutes at 37°C in a gyratory shaker water bath, setting 2-3, shaker model G76, New Brunswick Scientific. After incubation, the pH is raised to 8.0, 50 mg of trypsin (Worthington) are added with stirring, and the digest is incubated as above for 90 minutes.

The pH is adjusted to 6.5 and the digest is dispensed into 5/8" dialysis tubing and dialyzed against 14 liters of water.

The dialyzed solution (1,030 ml) is brought to 5% sodium acetate. Isopropanol probing indicates that the polysaccharide precipitates between 45-52% (vol/vol) isopropanol. Fractionating and precipitation are carried out as in the prior isopropanol step. The final precipitated polysaccharide is collected by siphoning off the supernatant. The precipitate is scraped into a Waring blender, triturated with 500 ml

-44-                    16129IA

of absolute ethanol, and washed onto a medium scinter glass funnel. The polysaccharide is further washed with 500 ml of absolute ethanol followed by 250 ml of acetone. Excess solvent is removed by suction, and the final product is dried _in vacuo_ over $CaSO_4$. Yield, 4.0 g.

## EXAMPLE 12

### PNEUMOCOCCAL POLYSACCHARIDE TYPE 17

A lyophilized culture of _Streptococcus pneumoniae_ type 17 is suspended in 1 ml of Difco Heart Infusion Broth (HIB broth) and 0.2 ml is spread on a rabbit blood agar plate. After 18 hours incubation at 37°C, the growth on the plate is resuspended in 5 ml of HIB broth and 1 ml of the suspension is transferred into 100 ml of rabbit blood liquid medium and incubated as a stationary culture for 18 hours at 37°C. After incubation the culture is examined microscopically and streaked on YED and HIB plates to check for purity. The 100 ml of culture is stored in the refrigerator at 4°C.

Ten ml of the culture of the incubated broth is used to inoculate 1 liter of inoculum medium in a 2-liter Erlenmeyer flask. The flask is incubated stationary for 19 hours at 37°C. The pH of the fermentation is controlled (6.4 - 7.0) by the addition of sodium bicarbonate (12%) until an O.D. of 3.68 is reached. The 1 liter fermentation utilizes 100 ml of sodium bicarbonate in reaching the above mentioned O.D. Before inoculating the next stage a sample is plated on YED and HIB plates to check purity. A sample is also examined microscopically and an agglutination test performed to check purity and type.

One liter of the fermentation broth from the previous step is used to inoculate a 14-liter fermentor containing 9 liters of production medium. The batch is incubated at 37°C with mild agitation (100 rpm) without air flow. After the final sodium hydroxide addition, the fermentation is continued one more hour and then terminated (total time 8 hours). The final O.D. before harvesting is 4.4. The pH is controlled during fermentation (6.2-7.0) by using 10% NaOH. A total of 695 ml of NaOH (10%) is utilized.

When the batch is terminated the culture is plated on YED and HIB plates, examined for purity by Gram stain, and identified as to type of the agglutination reaction. The broth is inactivated by harvesting into liquefied phenol (89%) to a final concentration of 1%. After 2 hours in phenol the broth is relased for recovery of product. A sample is removed and plated on HIB to check for viability.

After ethanol probing, 10 liters of the broth are brought to 50% ethanol (vol/vol) (95% denatured ethanol) by adding the alcohol with constant stirring at a flow rate of 100 ml/minute. The broth is then centrifuged in a Sharples centrifuge model T-1-P, 30,000 rpm.

To the clear effluent, 95% denatured ethanol is added to a final concentration of 57% (vol/vol) based on the original aqueous volume. The suspension is left overnight at room temperature during which time the precipitates settles. The crude polysaccharide is collected by siphoning the supernatant and the precipitate is dissolved in 1,000 ml of 3% sodium acetate.

Isopropanol probing indicates that the polysaccharide precipitates between 35-42% (vol/vol) of isopropanol at room temperature. Therefore, isopropanol is added dropwise with stirring on a magnetic stirrer, to a concentration of 35% (vol/vol). The suspension is cleared by centrifugation in a Beckman J21 at 14,000 rpm ($\sim$ 30,050 xg) for 20 minutes at 4°C. The clear supernatant is brought to 42% isopropanol, and the polysaccharide is collected by siphoning off the supernatant.

The precipitate is dissolved in 1,000 ml of water. The pH is adjusted to 7.0 and 0.203 g of $MgCl_2$ (1 mM), 8 mg of ribonuclease and 8 mg of deoxyribonuclease are added with stirring as in the previous step. The mixture is incubated for 90 minutes at 37°C in a gyratory shaker water bath. After incubation, 49 mg of trypsin (Worthington) are added. The digest is brought to pH 8.0 with 1N NaOH and incubated for 90 minutes as above.

The pH is adjusted to 6.5 and the digest is dispensed into 5/8" dialysis tubing and dialyzed against 14 liters of water.

980 ml are recovered and brought to 3% sodium acetate. Isopropanol probing indicates that the polysaccharide precipitates between 40-47% (vol/vol) isopropanol. Fractionation and precipitation are carried out as in the prior isopropanol step. The final precipitated polysaccharide is collected by siphoning off the supernatant. The polysaccharide is scraped into a Waring blender, triturated with 500 ml of absolute ethanol, and washed onto a medium scinter glass funnel. It is further washed with 500 ml of absolute ethanol, followed by 250 ml of acetone. Excess solvent is removed by suction, and the final product is dried _in vacuo_ over $CaSO_4$. Yield, 3.3 g.

EXAMPLE 13

Pneumococcal Polysaccharide Type 19

A culture of Streptococcus pneumoniae type 19 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1% (vol/vol) concentration to kill the bacteria.

An ethanol probe is run on a sample of the phenolized whole broth to determine the ethanol range of polysaccharide precipitation. The range for this lot is from 48 to 62% (vol/vol).

With agitation and at room temperature 5.54 liters of ethanol are added slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 4.25 liters of ethanol are added slowly to the supernatant liquid and the resulting mixture aged 4 hours with continuing agitation. The mixture is then centrigued at 15,000 rpm in 4" Sharples centrifuge. The supernatant liquid is discarded and the crude polysaccharide solids are suspended in 0.6 liter of 3% sodium acetate solution with the aid of a high shear mixer.  The pH is adjusted to 6.5 with glacial acetic acid and stirred for 4 hours to assure solubilization of the polysaccharide.

An isopropanol probe is run on a sample of the polysaccharide-containing liquid to determine optimum isopropanol range of polysaccharide precipitation. The range for this lot is from 35 to 45% (vol/vol).

With agitation 0.323 liter of isopropanol is added and the resulting mixture aged for 4 hours with continuing agitation.  The mixture is preclarified at 15,000 rpm in a 4" Sharples centrifuge. Clarification is completed in a K-2 ultracentrifuge at 25,000-30,000 rpm.  The solids are discarded.  With agitation an additional 0.168 liter of isopropanol is added to the supernatant liquid and the resulting mixture allowed to age 4 hours with agitation.  The crude polysaccharide precipitate is recovered by centrifuging at 15,000 rpm in a 4" Sharples centrifuge and discarding the supernatant liquid.

The crude polysaccharide is suspended with the aid of a high shear mixer in 0.3 liter of pH 7.0, 0.05 M phosphate buffer.  The pH is readjusted to 7.0 with glacial acetic acid and 60 mg $MgCl_2$, 0.3 mg ribonuclease and 0.3 mg deoxyribonuclease are added. The solution is digested with agitation at 37° for 60 minutes.  The pH is adjusted to 8.0 with 50% NaOH, 0.6 mg of trypsin are added, and the digestion is continued at 37° for 90 minutes.  The mixture is cooled to 20-25° and the pH adjusted to 6.5 with glacial acetic acid.

The solution is cooled to 0-5°, 180 g of ammonium sulfate are added, and the mixture aged at 0-5° for 4 hours. The precipitate which forms is collected by centrifuging at 15,000 rpm in a 4" Sharples centrifuge.  The supernatant liquid is discarded.

The precipitate is dissolved in 0.3 liter of pyrogen free water and the solution diafiltered in a Romicon hollow filter ultrafiltration membrane until the electrical conductivity of the permeate reaches a constant value. The reservoir is kept at constant volume with pyrogen free water. The retentate which contains the polysaccharide is drained from the system and the volume restored to 0.3 liter with system washes.

Sodium acetate, 9 g, is added to the solution of the polysaccharide-containing liquid and the pH is adjusted to pH 6.5 with glacial acetic acid. With agitation 0.6 liter of isopropanol is added and the resulting mixture stirred for 2 hours and settled for several hours until a gummy precipitate has separated to the bottom. The entire supernatant liquid is decanted and the precipitate is triturated in a Waring blender with about 40 ml of absolute ethanol until a white powder is obtained. The powder is recovered by filtering on a Buchner funnel (Whatman No. 2 paper) washing with 2 X 5 cc of absolute ethanol and 2 X 5 cc acetone. The washed powder is then dried at 27" Hg and room temperature for 12-16 hours to yield about 5.4 g of the purified polysaccharide.

EXAMPLE 14

PNEUMOCOCCAL POLYSACCHARIDE TYPE 20

A culture of Streptococcus pneumoniae type 20 (ATCC 6320) is suspended in 1 ml of Difco Heart Infusion Broth (HIB broth) and 0.2 ml is spread on a rabbit blood agar plate. After 18 hours incubation at 37°C, the growth on the plate is resuspended in 5 ml of HIB broth and 1 ml of the suspension is transferred into 100 ml of rabbit blood liquid medium and incubated as a stationary culture for 18 hours at 37°C. After incubation the culture is examined microscopically and streaked on YED and HIB plates to check for purity. The 100 ml of culture is stored in the refrigerator at 4°C.

Ten ml of the incubated culture is used to inoculate 1 liter of inoculum medium in a 2-liter Erlenmeyer flask. The flask is incubated stationary for 18 hours at 37°C. The pH of the fermentation is controlled (6.4-7.0) by the addition of bicarbonate (12%) until an O.D. of 2.8 is reached. The 1 liter fermentation utilizes 85 ml of sodium bicarbonate in reaching the above mentioned O.D. Before inoculating the next stage a sample is plated on YED and HIB plates to check purity. A sample is also examined microscopically and an agglutination test performed to check purity and type.

One liter of fermentation broth from the previous step is used to inoculate a 14-liter fermentor containing 9 liters of production medium. The batch is incubated at 37°C with mild agitation (100 rpm) without air flow. The course of the fermentation is monitored by O.D., pH and glucose determinations.

After the final sodium hydroxide addition, the fermentation is continued for two more hours and then terminated (total time 10 1/2 hours). The final O.D. before harvesting is 3.1. The pH is controlled during fermentation (6.2-7.0) by using 10% NaOH. A total of 700 ml of NaOH (10%) is utilized.

When the batch is terminated the culture is plated on YED and HIB plates, examined for purity by Gram stain, and identified as to type by the agglutination reaction. The broth is inactivated by harvesting into liquefied phenol (89%) to a final concentration of 1%. After 2 hours in phenol the broth is released for recovery of product. A sample is removed and plated on HIB to check for viability.

After ethanol probing, 95% denatured alcohol is added to 10 liters of the broth to bring the alcohol content to 53% (vol/vol). The alcohol is added with constant stirring at a flow rate of 100 ml/minute. The broth is then centrifuged in the Sharples Centrifuge, Model T-1-P, at 30,000 rpm.

To the clear effluent, additional 95% denatured alcohol is added to a final concentration of 65% vol/vol based on the original aqueous system. The insoluble material (which precipitates out immediately on addition of the alcohol) is collected by siphoning off the supernatant and by centrifugation in a Sorvall RC5 at 10,000 rpm (16,300 xg) in 500 ml cups at 23°C for ∿ 15 minutes.

The pellets are resuspended in 1,000 ml of 3% sodium acetate.

Isopropanol probing indicates that the polysaccharide precipitates between 44-52% (vol/vol) of isopropanol at room temperature. Therefore, isopropanol is added dropwise with stirring on a thermolyne stir plate, middle setting, to a 44% (vol/vol) level. The suspension is then centrifuged in a Sorvall

RC$_5$ in 250 ml cups at 14,000 rpm for 15 minutes at 23°C ($\sim$31,700 xg). The clear supernatant is diluted with isopropanol to a concentration of 52% (vol/vol). This results in the settling out of a gum which is collected by siphoning off the supernatant.

The gum is resuspended in 1,000 ml of pyrogen-free water. The pH is adjusted to 7.0. 203 Mg of MgCl$_2$ (1 mM), 4 mg of ribonuclease and 6 mg of deoxyribonuclease are added with stirring as in the previous step. This is incubated in a gyratory water bath for 1 hour at 37°C. After incubation, the pH is adjusted to 8.0 with NaOH, and 30 mg of trypsin are added with stirring as in the previous step. The mixture is incubated for 90 minutes as above.

The pH is adjusted to 7.0 and the digest is dispensed into 5/8" dialysis tubing and dialyzed against 14 liters of water with one change of water.

The dialyzed solution is diluted with 2 volumes of 95% denatured alcohol. The addition of 300 ml of an alcoholic electrolyte (1 volume of 20% sodium acetate, pH 6.5, plus 2 volumes of 95% denatured alcohol results in the precipitation of the polysaccharide. The polysaccharide is collected by centrifugation in a Beckman J21 in 500 ml cups at 10,000 rpm (17,680 xg) for 15 minutes at 23°C. The pellets are transferred into a Waring blender and are triturated with 500 ml of 95% denatured ethanol. The solid particles are washed onto a medium scinter glass funnel and are washed again with 500 ml of 95% denatured ethanol and 250 ml of acetone. After removal of excess solvent by suction, the polysaccharide is dried in vacuo over CaSO$_4$. The yield is 4.3 g.

## EXAMPLE 15

### Pneumococcal Polysaccharide Type 23

A culture of Streptococcus pneumoniae type 23 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1 % (vol/vol) concentration to kill the bacteria.

An ethanol probe is run on a sample of the phenolized whole broth to determine the ethanol range of polysaccharide precipitation. The range for this lot is from 48 to 62% (vol/vol).

With agitation and at room temperature 5.54 liters of ethanol are added slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 4.25 liters of ethanol are added slowly to the supernatant liquid and the resulting mixture aged 4 hours with continuing agitation. The mixture is then centrigued at 15,000 rpm in 4" Sharples centrifuge. The supernatant liquid is discarded and the crude polysaccharide solids are suspended in 0.6 liter of 3% sodium acetate solution with the aid of a high shear mixer. The pH is adjusted to 6.5 with glacial acetic acid and stirred for 4 hours to assure solubilization of the polysaccharide.

An isopropanol probe is run on a sample of the polysaccharide-containing liquid to determine optimum isopropanol range of polysaccharide precipitation. The range for this lot is from 33 to 48% (vol/vol).

With agitation 0.295 liter of isopropanol is added and the resulting mixture aged for 4 hours with continuing agitation. The mixture is preclarified at 15,000 rpm in a 4" Sharples centrifuge. Clarification is completed in a K-2 ultracentrifuge at 25,000-30,000 rpm. The solids are discarded. With agitation an additional 0.259 liter of isopropanol is added to the supernatant liquid and the resulting mixture allowed to age 4 hours with agitation. The crude polysaccharide precipitate is recovered by centrifuging at 15,000 rpm in a 4" Sharples centrifuge and discarding the supernatant liquid.

The crude polysaccharide is suspended with the aid of a high shear mixer in 0.6 liter of pH 7.0, 0.05 M phosphate buffer. The pH is readjusted to 7.0 with glacial acetic acid and 60 mg $MgCl_2$, 0.6 mg ribonuclease and 0.6 mg deoxyribonucelase are added. The solution is digested with agitation at 37° for 60 minutes. The pH is adjusted to 8.0 with 50% NaOH, 18 mg of trypsin are added, and the digestion is continued at 37° for an additional 90 minutes. The mixture is cooled to 20-25° and the pH adjusted to 6.5 with glacial acetic acid.

The solution is cooled to 0-5°, 5.1 g NaCl and 240 g of ammonium sulfate are added, and the mixture aged at 0-5° for 4 hours. The precipitate which forms is collected by centrifuging at 15,000 rpm in a 4" Sharples centrifuge. The supernatant liquid is discarded.

The precipitate is dissolved in 0.3 liter of pyrogen free water and the solution diafiltered in a Romicon hollow filter ultrafiltration membrane until the electrical conductivity of the permeate reaches a constant value. The reservoir is kept at constant volume with pyrogen free water. The retentate which contains the polysaccharide is drained from the system and the volume restored to 0.3 liter with system washes.

Sodium acetate, 9 g, is added to the solution of the polysaccharide-containing liquid and the pH is adjusted to pH 6.5 with glacial acetic acid. With agitation 0.6 liter of isopropanol is added and the resulting mixture stirred for 2 hours and settled for several hours until a gummy precipitate has separated to the bottom. The entire supernatant liquid is decanted and the precipitate is triturated in a Waring blender with about 40 ml of absolute ethanol until a white powder is obtained. The powder is recovered by filtering on a Buchner funnel (Whatman No. 2 paper) washing with 2 X 5 cc of absolute ethanol and 2 X 5 cc acetone. The washed powder is then dried at 27" Hg and room temperature for 12-16 hours to yield about 1.92 g of the purified polysaccharide.

## EXAMPLE 16

### Pneumococcal Polysaccharide Type 25

A culture of Streptococcus pneumoniae type 25 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1 % (vol/vol) concentration to kill the bacteria.

An ethanol probe is run on a sample of the phenolized whole broth to determine the ethanol range of polysaccharide precipitation. The range for this lot is from 52 to 65% (vol/vol).

With agitation and at room temperature 6.5 liters of ethanol are added slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 4.6 liters of ethanol are added slowly to the supernatant liquid and the resulting mixture aged 4 hours with continuing agitation. The mixture is then centrifuged at 15,000 rpm in 4" Sharples centrifuges. The supernatant liquid is discarded and the crude polysaccharide solids are suspended in 0.6 liters of 3% sodium acetate solution. The pH is adjusted to 6.5 with glacial acetic acid and the mixture stirred for 4 hours to assure solubilization of the polysaccharide.

An isopropanol probe is run on a sample of the polysaccharide-containing liquid to determine optimum isopropanol range of polysaccharide precipitation. The range for this lot is from 38 to 52% (vol/vol).

With agitation 0.37 liter of isopropanol is added and the resulting mixture aged for 4 hours with continuing agitation. The mixture is preclarified at 15,000 rpm in a 4" Sharples centrifuge. Clarification is completed in a K-2 ultracentrifuge at 25,000-30,000 rpm. The solids are discarded. With agitation an additional 0.3 liter of isopropanol is added to the supernatant liquid and the resulting mixture allowed to age 4 hours with agitation. The crude polysaccharide precipitate is recovered by centrifuging at 15,000 rpm in a 4" Sharples centrifuge and discarding the supernatant liquid.

The crude polysaccharide is suspended in 0.3 liter of pyrogen free water. The pH is adjusted to 7.0 with glacial acetic acid and 0.12 g. $MgCl_2$, 3.0 mg ribonuclease and 3.0 mg deoxyribonuclease are added. The solution is digested with agitation at 37° for 60 minutes. The pH is adjusted to 8.0 with 50% NaOH, 0.5 g of trypsin are added, and the digestion is continued at 37° for an additional 90 minutes. The mixture is cooled to 20-25° and the pH adjusted to 6.5 with glacial acetic acid.

The digested solution is diafiltered in a Romicon hollow filter ultrafiltration membrane until the electrical conductivity of the permeate reaches a constant value. The reservoir is kept at constant volume with pyrogen free water. The retentate which contains the polysaccharide is drained from the system and the volume restored to 0.3 liter with system washes.

The pH of the polysaccharide-containing liquid is adjusted to pH 6.5 with glacial acetic acid and an isopropanol probe is run on a sample of the liquid to determine the optimum isopropanol range of polysaccharide precipitation. With this lot the range is from 38-47% (vol/vol). With agitation 0.18 liter of isopropanol is added and the resulting mixture aged for 4 hours. The mixture is preclarified in a 4" Sharples centrifuge at 15,000 rpm. Clarification is completed in a K-2 ultracentrifuge at 25,000-30,000 rpm. The solids are discarded and an additional 0.082 liter of isopropanol is charged to the K-2 supernatant and the resulting mixture aged for 4 hours with agitation. The crude polysaccharide precipitate is recovered by centrifugation at 15,000 rpm in a 4" Sharples centrifuge and discarding the supernatant liquid.

The precipitate is triturated in a Waring blender with about 40 ml of absolute ethanol until a white powder is obtained. The powder is recovered by filtering on a Buchner funnel (Whatman No. 2 paper) washing with 2 X 5 cc of absolute ethanol and 2 X 5 cc acetone. The washed powder is then dried at 27" Hg and room temperature for 12-16 hours to yield about 2.5 g of the purified polysaccharide.

EXAMPLE 17

Pneumococcal Polysaccharide Type 51

A culture of Streptococcus pneumonia type 51 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of NaHCO$_3$ at 2 hour intervals to neutralize the medium. Phenol is then added to the medium to a 1% (vol/vol) concentration to kill the bacteria.

An ethanol probe is run on a sample of the phenolized whole broth to determine the ethanol range of polysaccharide precipitation. The range for this lot is from 50 to 70% (vol/vol).

With agitation and at room temperature 6 liters of ethanol are added slowly to 6 liters of the phenolized whole broth and the resulting mixture aged 4 hours with continuing agitation. The mixture is then centrifuged at 15,000 rpm in a 4" Sharples centrifuge. The solids are discarded.

With agitation an additional 8 liters of ethanol are added slowly to the supernatant liquid and the resulting mixture aged 4 hours with continuing agitation. The mixture is then centrigued at 15,000 rpm in 4" Sharples centrifuge. The supernatant liquid is discarded and the crude polysaccharide solids are suspended in 0.6 liter of 3% sodium acetate solution with the aid of a high shear mixer. The pH is adjusted to 6.5 with glacial acetic acid and stirred for 4 hours to assure solubilization of the polysaccharide.

An isopropanol probe is run on a sample of the polysaccharide-containing liquid to determine optimum isopropanol range of polysaccharide precipitation. The range for this lot is from 42 to 57% (vol/vol).

With agitation 0.434 liter of isopropanol is added slowly and the resulting mixture aged for 4 hours with continuing agitation. The mixture is preclarified at 15,000 rpm in a 4" Sharples centrifuge. Clarification is completed in a K-2 ultracentrifuge at 25,000-30,000 rpm. The solids are discarded. With agitation an additional 0.361 liter of isopropanol is added slowly to the supernatant liquid and the resulting mixture allowed to age 4 hours with agitation. The crude polysaccharide precipitate is recovered by centrifuging at 15,000 rpm in a 4" Sharples centrifuge and discarding the supernatant liquid.

The crude polysaccharide is suspended with the aid of a high shear mixer in 0.3 liter of pH 7.0, 0.05 M phosphate buffer. The pH is readjusted to 7.0 with glacial acetic acid and 60 mg $MgCl_2$, 0.6 mg ribonuclease and 0.6 mg deoxyribonuclease are added. The solution is digested with agitation at 37° for 60 minutes. The pH is adjusted to 8.0 with 50% NaOH, 18 mg of trypsin are added, and the digestion is continued at 37° for an additional 90 minutes. The mixture is cooled to 20-25° and the pH adjusted to 6.5 with glacial acetic acid.

The digested solution is diafiltered in a Romicon hollow filter ultrafiltration membrane until the electrical conductivity of the permeate reaches a constant value. The reservoir is kept at constant volume with pyrogen free water. The retentate which contains the polysaccharide is drained from the system and the volume restored to 0.3 liter with system washes.

Sodium acetate, 9 g, is added to the solution of the polysaccharide-containing liquid and the pH is adjusted to 6.5 with glacial acetic acid. With agitation 0.6 liter of isopropanol are added and the resulting mixture stirred for 15 minutes and settled for several hours until a gummy precipitate has separated to the bottom. The entire supernatant liquid is decanted and the precipitate is triturated in a Waring blender with about 40 ml of absolute ethanol until a white powder is obtained. The powder is recovered by filtering on a Buchner funnel (Whatman No. 2 paper) washing with 2 X 5 cc of absolute ethanol and 2 X 5 cc acetone. The washed powder is then dried at 27" Hg and room temperature for 12-16 hours to yield about 3.33 g of the purified polysaccharide.

## EXAMPLE 18

### Pneumococcal Polysaccharide Type 56

A culture of Streptococcus pneumoniae type 56 is grown on blood agar for 16 hours at 37°. The cells are collected and placed in 250 ml of blood broth for 16 hours at 37°. A 5-10 ml aliquot of the blood broth growth is used to inoculate a 2 liter flask containing beef extract nutrient medium. The culture is grown for 16 hours at 37° with addition of $NaHCO_3$ at 2 hour intervals to neutralize the medium. A 14 liter flask containing beef extract nutrient medium is inoculated with the contents from the 2 liter flask and grown for 16 hours at 37° with addition of $NaHCO_3$ at 2 hour intervals to neutralize the medium. Phenol is then added to the medium to a 1 % (vol/vol) concentration to kill the bacteria.

An ethanol probe is run on a sample of the phenolized whole broth to determine the ethanol range of polysaccharide precipitation. The range for this lot is from 52 to 67% (vol/vol).

With agitation and at room temperature 6.5 liters of ethanol are added slowly to 6 liters of the phenolized whole broth and the resulting mixture aged 4 hours with continuing agitation. The mixture is then centrifuged at 15,000 rpm in 4" Sharples centrifuges. The solids are discarded.

With agitation, an additional 5.68 liters of ethanol are added slowly to the supernatant liquid and the resulting mixture aged 4 hours with continuing

0002404

agitation. The mixture is then centrigued at 15,000 rpm in 4" Sharples centrifuge.  The supernatant liquid is discarded and the crude polysaccharide solids are suspended in 0.6 liter of 3% sodium acetate solution with the aid of a high shear mixer.  The pH is adjusted to 6.5 with glacial acetic acid and stirred for 4 hours to assure solubilization of the polysaccharide.

An isopropanol probe is run on a sample of the polysaccharide-containing liquid to determine optimum isopropanol range of polysaccharide precipitation.  The range for this lot is from 38 to 50% (vol/vol).

With agitation 0.368 liter of isopropanol is added and the resulting mixture aged for 4 hours with continuing agitation.  The mixture is preclarified at 15,000 rpm in a 4" Sharples centrifuge. Clarification is completed in a K-2 ultracentrifuge at 25,000-30,000 rpm.  The solids are discarded. With agitation, an additional 0.232 liter of isopropanol is added to the supernatant liquid and the resulting mixture allowed to age 4 hours with agitation.  The crude polysaccharide precipitate is recovered by centrifuging at 15,000 rpm in a 4" Sharples centrifuge and discarding the supernatant liquid.

The crude polysaccharide is suspended with the aid of a high shear mixer in 0.3 liter of pH 7.0, 0.05 M phosphate buffer.  The pH is adjusted to 7.0 with glacial acetic acid and 60 mg MgCl$_2$, 0.6 mg ribonuclease and 0.6 mg deoxyribonuclease are added. The resulting solution is digested with agitation at 37° for 60 minutes.  The pH is adjusted to 8.0 with 50% NaOH, 18 mg of trypsin are added, and the digestion is continued at 37° for an additional 90 minutes.  The mixture is cooled to 20-25° and the pH adjusted to 6.5 with glacial acetic acid.

The solution is cooled to 0-5°, 2.55 g of NaCl and 120 g sulfate are added, and the mixture aged at 0-5° for 4 hours. The precipitate which forms is collected by centrifuging at 20,000-25,000 rpm in the K-2 ultra centrifuge. The supernatant liquid is discarded.

The precipitate is dissolved in 0.3 liter of pyrogen free water and the solution diafiltered in a Romicon hollow filter ultrafiltration membrane until the electrical conductivity of the permeate reaches a constant value. The reservoir is kept at constant volume with pyrogen free water. The retentate which contains the polysaccharide is drained from the system and the volume restored to 0.3 liter with system washes.

Sodium acetate, 9 g, is added to the solution of the polysaccharide-containing liquid and the pH is adjusted to pH 6.5 with glacial acetic acid. With agitation 0.6 liter of isopropanol is added and the resulting mixture stirred for 2 hours and settled for several hours until a gummy precipitate has separated to the bottom. The entire supernatant liquid is decanted and the precipitate is triturated in a Waring blender with about 40 ml of absolute ethanol until a white powder is obtained. The powder is recovered by filtering on a Buchner funnel (Whatman No. 2 paper) washing with 2 X 5 cc of absolute ethanol and 2 X 5 cc acetone. The washed powder is then dried at 27" Hg and room temperature for 12-16 hours to yield about 2.04 g of the purified polysaccharide.

EXAMPLE 19

PNEUMOCOCCAL POLYSACCHARIDE TYPE 57

A culture of Streptococcus pneumoniae type 57 is suspended in 1 ml of Difco Heart Infusion Broth (HIB broth) and 0.2 ml is spread on a rabbit blood agar plate. After 18 hours incubation at 37°C, the growth on the plate is resuspended in 5 ml of HIB broth and 1 ml of the suspension is transferred into 100 ml of rabbit blood liquid medium and incubated as a stationary culture for 18 hours at 37°C. After incubation the culture is examined microscopically and streaked on YED and HIB plates to check for purity. The 100 ml of culture is stored in the refrigerator at 4°C.

Ten ml of the incubated culture is used to inoculate 1 liter of inoculum medium in a 2-liter Erlenmeyer flask. The flask is incubated stationary for 18 hours at 37°C. The pH of the fermentation is controlled (6.4-7.0) by the addition of sodium bicarbonate (12%) until an O.D. of 2.0 is reached. The 1 liter fermentation utilizes 100 ml of sodium bicarbonate in reaching the above mentioned O.D. Before inoculating the next stage a sample is plated on YED and HIB plates to check purity. A sample is also examined microscopically and an agglutination test performed to check purity and type.

One liter of fermentation broth from the previous step is used to inoculate a 14-liter fermentor containing 9 liters of production medium. The batch is incubated at 37°C with mild agitation (100 rpm) without air flow. The course of the fermentation is monitored by O.D., pH and glucose determinations.

After the final sodium hydroxide addition, the fermentation is continued for one more hour and then terminated (total time 10 1/2 hours). The final O.D. before harvesting is 5.0. The pH is controlled during fermentation (6.2-7.0) by using 10% NaOH. A total of 655 ml of NaOH (10%) is utilized.

When the batch is terminated the culture is plated on YED and HIB plates, examined for purity by Gram stain, and identified as to type by the agglutination reaction. The broth is inactivated by harvesting into liquefied phenol (89%) to a final concentration of 1%. After 2 hours in phenol the broth is released for recovery of product. A sample is removed and plated on HIB to check for viability.

After ethanol probing, 10 liters of the broth is brought to 50% (vol/vol) ethanol by addition of 95% denatured alcohol at a flow rate of 100 ml/minute with constant stirring. The broth is then cleared by centrifugation in the Sharples centrifuge at 40,000 rpm.

To the clear effluent, additional 95% denatured alcohol is added to 65% (vol/vol) based on the original aqueous system. The precipitate is collected by siphoning off most of the supernatant and by centrifuging the remaining supernatant plus crude polysaccharide in a Sorvall RC5 at 6,000 rpm (6089 xg) for 20 minutes at 5°C.

The pellets are resuspended in 1,000 ml of 3% sodium acetate with the aid of a Waring blender at low speed. The pH is adjusted to 6.5

Isopropanol probing indicates that the polysaccharide precipitates between 30-42% (vol/vol) of isopropanol at room temperature. Therefore, isopropanol is added dropwise with stirring on a thermolyne stir plate, middle setting, to a concentration of 30% (vol/vol). The suspension is then
cleared by centrifugation in a Beckman J21 at 14,000
rpm (30,050 xg) at 15°C for 20 minutes. The clear
supernatant is diluted with isopropanol to a final
concentration of 42%. This results in the settling
out of a precipitate which is collected by centrifugation in a Beckman J21 at 6,000 rpm for 15
minutes at 5°C (6,370 xg).

The precipitated polysaccharide is
resuspended in 1,000 ml of water and the pH is
adjusted to 7.0 with concentrated acetic acid.
203 Mg of $MgCl_2$ (1 mM), 10 mg of deoxyribonuclease
and 15 mg of ribonuclease are added with stirring. The
mixture is incubated in a gyratory water bath, for
80 minutes at 37°C. After incubation, the pH is adjusted to 8.0 with 1 N NaOH, and 30 mg of trypsin is
added. The digest is incubated as above.

The pH is adjusted to 7.0 and the digest is
dispensed into 1 1/8" dialysis tubing and dialyzed
against 14 liters of water with one change of water.

Approximately 1,000 ml are recovered and
brought to 3% sodium acetate. Isopropanol probing
indicates that the purified polysaccharide precipitates
between 40-50% (vol/vol) isopropanol, at room temperature.
The precipitate is performed as in the prior isopropanol
step. The polysaccharide which adheres to the bottom
of the glass beaker is collected by siphoning off
the final supernatant. It is triturated in a Waring
blender at high speed with 500 ml of absolute ethanol
and is poured onto a medium sinter glass funnel. The
polysaccharide is washed with 500 ml of ethanol followed
by 250 ml of acetone. Excess solvent is removed by
suction and the polysaccharide is dried in vacuo over
$CaSO_4$. The yield 3.7 g.

EXAMPLE 20

PNEUMOCOCCAL POLYSACCHARIDE TYPE 72

A culture of Streptococcus pneumoniae type 72 is suspended in 1 ml of Difco Heart Infusion Broth (HIB broth) and 0.2 ml is spread on a rabbit blood agar plate. After 18 hours incubation at 37°C, the growth on the plate is resuspended in 5 ml of HIB broth and 1 ml of the suspension is transferred into 100 ml of rabbit blood liquid medium and incubated as a stationary culture for 18 hours at 37°C. After incubation the culture is examined microscopically and streaked on YED and HIB plates to check for purity. The 100 ml of culture is stored in the refrigerator at 4°C.

Ten ml of the incubation culture is used to inoculate 1 liter of inoculum medium in a 2-liter Erlenmeyer flask. The flask is incubated stationary for 19 hours at 37°C. The pH of the fermentation is controlled (6.4-7.0) by the addition of sodium bicarbonate (12%) until an O.D. of 1.24 is reached. The 1 liter fermentation utilizes 55 ml of sodium bicarbonate in reaching the above mentioned O.D. Before inoculating the next stage a sample is plated on YED and HIB plates to check purity. A sample is also examined microscopically and an agglutination test performed to check purity and type.

One liter of fermentation broth from the previous step is used to inoculate a 14-liter fermentor containing 9 liters of production medium. The batch is incubated at 37°C with mild agitation (100 rpm) without air flow. The course of the fermentation is monitored by O.D., pH and glucose determinations.

When the O.D. determinations are similar for a two-hour period the fermenation is terminated (total incubation time 10 hours). The final O.D. before harvesting is 3.68. The pH is controlled during fermentation (6.2-7.0) by using 10% NaOH. A total of 600 ml of NaOH (10%) is utilized.

When the batch is terminated the culture is plated on YED and HIB plates, examined for purity by Gram stain, and identified as to type by the agglutination reaction. The broth is inactivated by harvesting into liquefied phenol (89%) to a final concentration of 1%. After 2 hours in phenol the broth is released for recovery of product. A sample is removed and plated on HIB to check for viability.

After ethanol probing, the broth is brought to 42% (vol/vol) ethanol by addition of 95% denatured ethanol. The alcohol is added slowly with constant stirring at a flow rate of 100 ml/minute. The broth is then centrifuged in the Sharples Centrifuge, Model T-1-P, at 30,000 rpm.

To the clear effluent, 95% denatured ethanol is added to a final concentration of 57% (vol/vol) based on the original aqueous system. The insoluble material (which precipiates out immediately with the addition of the alcohol) is collected by siphoning off the supernatant and by centrifugation in a Beckman J21 at 9,000 rpm ( $\sim$ 14,000 xg) at 23°C for 20 minutes.

The pellets are resuspended in 1,000 ml of 3% sodium acetate, pH 6.5.

Isopropanol probing indicates that the polysaccharide precipitates between 30-45% (vol/vol) of isopropanol at room temperature. Therefore, isopropanol is added dropwise with stirring to a 30% (vol/vol) level. The suspension is then centrifuged in a Beckman J21 in 250 ml cups at 10,000 rpm at 23°C (15,380 xg). The clear supernatant is diluted with isopropanol to a concentration of 45% (vol/vol). This results in the settling out of a semi-liquid gum which is collected by siphoning off the supernatant.

The gum is resuspended in 1,000 ml of pyrogen-free water. The pH is adjusted to 7.0. 203 Mg of $MgCl_2$ (1 mM), 4 mg of ribonuclease and 6 mg of deoxyribonuclease are added with stirring. The mixture is incubated in a gyratory water bath for 1 hour at 37°C. After incubation, the pH is adjusted to 8.0 with NaOH, and 30 mg of trypsin are added with stirring. The mixture is incubated for 90 minutes as above.

The pH is adjusted to 6.5 and the digest is dispensed into 5/8" dialysis tubing and dialyzed against 14 liters of water with one change of water.

The dialyzed solution (950 ml) is diluted with 2 volumes of 95% denatured ethanol. The addition of 150 ml of an alcoholic electrolyte (1 volume of 20% sodium acetate, pH 6.5, plus 2 volumes of 95% denatured ethanol results in the precipitation of the polysaccharide. The polysaccharide is collected by siphoning off the supernatant; and the "mat" is transferred into a Waring blender and triturated with 500 ml of 95% denatured ethanol. The solid particles are washed onto a medium scinter glass funnel and are washed again with 500 ml of 95% denatured ethanol and 250 ml of acetone. After removal of excess solvent by suction, the polysaccharide is dried _in vacuo_ over $CaSO_4$. The yield is 7.3 g.

EXAMPLE 21

PNEUMOCOCCAL POLYSACCHARIDE TYPE 73

A lyophilized culture of Streptococcus pneumoniae type 72 is suspended in 1 ml of Difco Heart Infusion Broth (HIB broth) and 0.2 ml is spread on a rabbit blood agar plate. After 18 hours incubation at 37°C, the growth on the plate is resuspended in 5 ml of HIB broth and 1 ml of the suspension is transferred into 100 ml of rabbit blood liquid medium and incubated as a stationary culture for 18 hours at 37°C. After incubation the culture is examined microscopically and streaked on YED and HIB plates to check for purity. The 100 ml of culture is stored in the refrigerator at 4°C.

Ten ml of the incubation culture is used to inoculate 1 liter of inoculum medium in a 2-liter Erlenmeyer flask. The flask is incubated stationary for 16 hours at 37°C. The pH of the fermentation is controlled (6.4-7.0) by the addition of sodium bicarbonate (12%) until an O.D. of 1.4 is reached. The 1 liter fermentation utilizes 120 ml of sodium bicarbonate in reaching the above mentioned O.D. Before inoculating the next stage a sample is plated on YED and HIB plates to check purity. A sample is also examined microscopically and an agglutination test performed to check purity and type.

One liter of fermentation broth from the previous step is used to inoculate a 14-liter fermentor containing 9 liters of production medium. The batch is incubated at 37°C with mild agitation (100 rpm) without air flow. The course of the fermentation is monitored by O.D., pH and glucose determinations.

When the O.D. determinations are similar for a
two-hour period the fermenation is terminated
(17 hours total incubation). The final O.D. before
harvesting is 4.48. The pH is controlled during
fermentation (6.2-7.0) by using 10% NaOH.
A total of 665 ml of NaOH (10%) is utilized.

When the batch is terminated the culture
is plated on YED and HIB plates, examined for purity
by Gram stain, and identified as to type by the
agglutination reaction. The broth is inactivated by
harvesting into liquefied phenol (89%) to a final
concentration of 1%. After 2 hours in phenol the
broth is released for recovery of product. A sample
is removed and plated on HIB to check for viability.

After ethanol probing, the broth is brought
to 40% (vol/vol) alcohol by addition of 95% denatured
ethanol. The alcohol is added slowly with constant
stirring at a flow rate of 100 ml/minute. The broth is
then centrifuged in the Sharples Centrifuge, Model
T-1-P, at 30,000 rpm.

To the clear effluent, 95% denatured
ethanol is added to a final concentration of 60%
(vol/vol) based on the original aqueous system. The
suspension is left overnight at room temperature
during which time the precipitate settles. THe
insoluble material (crude polysaccharide) is
collected by siphoning off the clear supernatant
and centrifugation in the Beckman J21 at 5,000 rpm
( ∼ 4,000 xg) for 10 minutes at 23°C in 500 ml cups.

The pelleted material is suspended in 1,000
ml of 3% sodium acetate buffer, pH 6.5, with stirring
on a thermolyne stir plate, middle setting.

Isopropanol probing indicates that the
polysaccharide precipitates between 20-42% (vol/vol)
of isopropanol at room temperature. Therefore,
isopropanol is added dropwise with stirring to a to a concentration of 20% (vol/vol), and the suspension is centrifuged in a Beckman J21 at 10,000 rpm
(15,380 xg) in 250 ml cups at 23°C.  The clear supernatant is further diluted with isopropanol to a concentration of 42% (vol/vol).  This results in the
settling out of a "semi-liquid gum" which is collected
by siphoning off the supernatant and by centrifugation
in a Beckman J21 at 6,000 rpm ($\sim$5,520 xg) in 250 ml
cups at 23°C.

The pellet from the previous step is dissolved
in 1,400 ml of 1% sodium acetate buffer.  The pH is
adjusted to 7.0, 284 mg of $MgCl_2$ (1mM), 1 mg of
ribonuclease and 1 mg of deoxyribonuclease are added
with stirring as in the previous step.  The mixture
is incubated in a gyratory shaker water bath for one
hour at 37°C.  After incubation the pH is adjusted to
8.0 with 1m NaOH, and 30 mg of trypsin are added with
stirring.  The mixture is incubated as above for 90
minutes.

The pH is adjusted to 6.5 and the digest
is dispensed into 5/8" dialysis tubing and dialyzed
against 14 liters of water (changed three times).

The dialyzed solution (1,680 ml) is diluted
with 2 volumes of 95% denatured ethanol.  The
addition of 150 ml of an alcoholic electrolyte (1
volume of 20% sodium acetate, plus 2 volumes of
95% denatured ethanol results in the immediate
flocculation of the polysaccharide.  The poly-
saccharide is collected by centrifugation in a Beckman
J21, 250 ml cups, for 15 minutes at 10,000 rpm
(15,380 xg) at 23°C.  The pellets are transferred into
a Waring blender and are triturated with 500 ml of
95% denatured ethanol.  The solid particles are
washed onto a medium scinter glass funnel with 500 ml
of 95% denatured ethanol followed by 250 ml of acetone.

After removal of excess solvent by suction, the polysaccharide is dried $\underline{in}$ $\underline{vacuo}$ over $CaSO_4$. The yield is 7.9 g.

## EXAMPLE 22

A polyvalent vaccine is prepared by adding under aseptic conditions at room temperature the following amounts of capsular polysaccharide to 3,160 ml of pyrogen free saline (0.85%). The polysaccharides are added individually at 15 second intervals while stirring continuously in a blendor. After the last polysaccharide is added, stirring is continued for another 2-3 minutes.

| Type | Amount (Gram) |
|------|---------------|
| 1 | 0.40025 |
| 2 | 0.37455 |
| 3 | 0.35600 |
| 4 | 0.34730 |
| 5 | 0.34875 |
| 8 | 0.34965 |
| 9 | 0.34165 |
| 12 | 0.36610 |
| 14 | 0.33555 |
| 19 | 0.37185 |
| 23 | 0.35615 |
| 25 | 0.36040 |
| 51 | 0.34070 |
| 56 | 0.34820 |
|    | 4.9979 |

The mixture is then sterile filtered and packaged in vials, 6 ml (10 doses) per vial.

0002404

WHAT IS CLAIMED IS:

1. A vaccine effective against bacterial pneumonia comprising a physiologically acceptable medium and a member selected from

a purified capsular polysaccharide of Streptococcus pneumoniae type 19 containing from about 15 to about 20% L-rhamnose, from about 5.5 to about 8% D-glucose, and from about 8 to about 11% 2-acetamido-2-deoxy-D-mannose,

or a purified capsular polysaccharide of Streptococcus pneumoniae type 23 containing from about 31 to about 38% L-rhamnose, from about 15 to about 18% D-galactose and from about 16 to about 18% D-glucose,

or a purified capsular polysaccharide of Streptococcus pneumoniae type 25 containing from about 15 to about 19% galactose, from about 15 to about 19% glucosamine, from about 7 to about 9% galactosamine, from about 5 to about 7% of uronic acid, from about 2 to about 4% fucose, from about 2 to 4% mannose and from about 1.8 to about 4% D-glucose,

or a purified capsular polysaccharide of Streptococcus pneumoniae type 72 containing from about 27 to about 32% glucosamine, from about 17 to about 22% D-galactose, from about 11.5 to about 16.5% galactosamine, from about 5.5 to about 8% L-rhamnose, from about 3 to about 6.5% fucose, and from about 2.7 to about 4.8% mannose,

or a purified capsular polysaccharide of Streptococcus pneumoniae type 73 containing from about 29 to about 34% glucosamine, from about 14 to about 19% D-galactose, from about 12 to about 15% galactosamine, from about 2 to about 5% mannose, and from about 1.8 to about 3.5% fucose.

0002404

2.  A method of preparing a purified capsular polysaccharide of Streptococcus pneumoniae comprising adding a predetermined quantity of a water-miscible alcohol to a liquid medium containing the polysaccharide thereby precipitating impurities, separating the impurities from the liquid medium, adding a predetermined quantity of a water-miscible alcohol to the liquid medium thereby precipitating the polysaccharide, resuspending the polysaccharide in liquid medium, and removing proteins and nucleic acids from the polysaccharide.

3.  A method according to claim 2 wherein the liquid medium is a fermentation broth.

4.  A method according to claim 2 wherein the water-miscible alcohol has from 2 to 3 carbon atoms.

5.  A method according to claim 2 wherein proteins and nucleic acids are removed by treatment with trypsin and nucleases.

6.  A method according to claim 2 wherein proteins and nucleic acids are removed by treatment with a cationic surfactant.

7.  A method according to claim 6 wherein the cationic surfactant is cetrimonium bromide.

8.  A method according to claim 5 wherein protein fragments and nucleic acid fragments are removed by diafiltration.

9.  A method according to claim 6 wherein protein fragments and nucleic acid fragments are removed by diafiltration.

10.  A vaccine comprising the polysaccharide of claim 1 and a physiologically acceptable medium.

European Patent Office

**EUROPEAN SEARCH REPORT**

| DOCUMENTS CONSIDERED TO BE RELEVANT | | CLASSIFICATION OF THE APPLICATION (Int. Cl.²) |
|---|---|---|
| Citation of document with indication, where appropriate, of relevant passages | Relevant to claim | |
| CHEMICAL ABSTRACTS, vol. 61, November 9, 1964, no. 10, ref. 12524h Columbus Ohio USA R. AUSTRIAN et al. "Pneumococcal bacteremia with especial reference to bacteremic pneumococcal pneumonia". & Ann Internal Med. 60,759-76 (1964) * Abstract * | 1-10 | A 61 K 39/02 C 12 D 13/04 C 12 K 5/00 |
| | | TECHNICAL FIELDS SEARCHED (Int. Cl.²) |
| UNLISTED DRUGS, vol. 28, no. 12, December 1976, & Clin. Res, 24: 348A, April 1976. * Page 205, left-hand column, point d, "5558" * | 1-10 | A 61 K 39/02 C 12 D 13/04 C 12 K 5/00 |
| C.A. WILLIAMS and M.W. CHASE, Ed., Methods in Immunology and Immunochemistry, Volume I: Preparation of Antigens and Antibodies, Academic Press, New York and London, 1967. * Pages 52-60 and page 42, lines 11-15 * | 1-10 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 26-02-1979 | RIJCKEBOSCH |

EPO Form 1503.1  06.78